# EUROPÄISCHE PATENTSCHRIFT

(11) **EP 3 445 067 B1**
(45) Veröffentlichungstag und Bekanntmachung des Hinweises auf die Patenterteilung: **25.11.2020**
(21) Anmeldenummer: 18186743.3
(22) Anmeldetag: 01.08.2018
(51) Int. Cl.: H04R 25/00

(54) **VERFAHREN ZUM BETRIEB EINES HÖRGERÄTS UND HÖRGERÄT**
HEARING AID AND METHOD FOR OPERATING A HEARING AID
PROCÉDÉ DE FONCTIONNEMENT D'UN APPAREIL DE CORRECTION ADDITIVE ET APPAREIL DE CORRECTION ADDITIVE

(30) Priorität: 14.08.2017 DE 102017214164
(43) Veröffentlichungstag der Anmeldung: 20.02.2019
(73) Patentinhaber: Sivantos Pte. Ltd., Singapore 539775 (SG)
(72) Erfinder: HANNEMANN, Ronny, 91054 Buckenhof (DE); STRAUSS, Daniel J., 66129 Saarbrücken (DE); CORONA-STRAUSS, Farah I., 66129 Saarbrücken (DE)
(74) Vertreter: FDST Patentanwälte

(56) Entgegenhaltungen:
- US-A1- 2007 112 277
- US-A1- 2010 160 714
- US-A1- 2012 245 655
- US-A1- 2016 119 726
- US-A1- 2016 157 030

## Beschreibung

Die Erfindung betrifft ein Verfahren zum Betrieb eines Hörgeräts sowie ein entsprechendes Hörgerät.

Ein Hörgerät dient allgemein der Wiedergabe eines Schallsignals in Richtung eines Ohrs eines Anwenders. Das Hörgerät wird hierzu im oder am Ohr getragen und weist einen Hörer auf, über welchen eine Schallausgabe erfolgt. Im Speziellen dient ein Hörgerät der Versorgung eines hörgeschädigten Anwenders. Ein solches Hörgerät weist üblicherweise eine Anzahl an Mikrofonen auf, zur Aufnahme von Schallsignalen aus der Umgebung, und eine Signalverarbeitungseinheit, welche die aufgenommenen Schallsignale geeignet modifiziert, insbesondere verstärkt, und dann an den Hörer zur Ausgabe weiterleitet.

Der Nutzen eines solchen Hörgeräts für den Anwender hängt wesentlich von der Fähigkeit des Hörgeräts ab, die Schallsignale derart auszugeben, dass diese den Bedürfnissen des Anwenders in einer konkreten Situation möglichst optimal entsprechen. Zunächst wird daher im Rahmen einer Fitting Session das Hörgerät an das individuelle Hörprofil des Anwenders angepasst. Dies geschieht über die Einstellung einer Anzahl an Betriebsparametern des Hörgeräts, welche dann dessen Verhalten im Normalbetrieb des Hörgeräts, d.h. bei der Anwendung im Alltag, definieren. Darüber hinaus werden im Normalbetrieb idealerweise die sich verändernde Situation und insbesondere die veränderlichen Umgebungsbedingungen mit berücksichtigt, z.B. ob der Anwender sich in einer leisen oder lauten Umgebung aufhält, ob viele oder wenige Schallquellen vorhanden sind oder welcher Art eine Schallquelle ist. Hierzu werden verschiedene Betriebsmodi bereitgestellt, mit unterschiedlichen Einstellungen für die Betriebsparameter. Ein jeweiliger Betriebsmodus wird dann manuell vom Anwender eingestellt oder automatisch vom Hörgerät selbst.

Für eine automatische Einstellung werden zusätzliche Sensoren herangezogen oder die von den Mikrofonen erzeugten Mikrofonsignale analysiert. Dadurch werden zusätzliche Erkenntnisse über die zu einem gegebenen Zeitpunkt herrschenden Umgebungsbedingungen gewonnen. Diese Erkenntnisse fließen dann in ein Anpassungskonzept der Betriebsparameter des Hörgeräts im Normalbetrieb ein. Beispielsweise können mittels eines Klassifikators bestimmte Strukturen in den Mikrofonsignalen erkannt werden und so bestimmte vordefinierte Situationen erkannt werden, z.B. "Sprache", "Musik" oder "Hintergrundgeräusche". Als Reaktion auf die Erkennung einer bestimmten Umgebung wird dann ein entsprechend geeigneter Betriebsmodus mit geeigneten Betriebsparametern eingestellt. Zusätzliche Sensoren können ebenso hierfür herangezogen werden, denkbar sind z.B. Beschleunigungssensoren, mittels welchen eine Kopfbewegung erkannt wird, woraus dann geschlossen wird, dass der Anwender in einer anderen Richtung hören will.

Die genannten Lösungen zur Verbesserung der Einstellung des Hörgeräts im Betrieb sind lediglich Beispiele aus einer Vielzahl an Möglichkeiten. Gemeinsam ist diesen Lösungsansätzen jedoch, dass sie von Sensoren abhängig sind, welche prinzipbedingt externe Einflüsse erfassen und daher entsprechend fehleranfällig sind, vor allem anfällig für Fehlinterpretationen bezüglich dem Willen des Anwenders. Wird beispielsweise in einer Richtung ein plötzlich neu auftauchendes Geräusch gemessen, bedeutet dies noch nicht zwangsläufig, dass der Anwender die Hörrichtung ändern will, denn es könnte sich um eine unbedeutende Ablenkung handeln. Die tatsächliche Intention des Anwenders bleibt prinzipbedingt unbekannt. Eine andere Idee ist daher die Messung von Hirnströmen und die Erzeugung eines EEG (Elektroenzephalogramm). Die mittels EEG gemessenen Hirnströme sind die Summe verschiedener Nervensignale. Da die Hirnströme des Anwenders selbst als Antwort auf die gerade vorliegende Situation erzeugt werden, kann ein gewisses Maß an Intention bereits vorausgesetzt werden, sodass die Hirnströme als Signale mit hoher Wahrscheinlichkeit relevant bezüglich der aktuellen Situation sind. Zur Messung der Hirnströme wird ein Elektrodenarray mit einer Vielzahl an Elektroden über die Kopfhaut des Anwenders verteilt angebracht. Bestimmte Reaktionen des Anwenders erzeugen dann ein bestimmtes Muster an Hirnströmen, sodass anhand der Erkennung dieser Muster dann eine gezielte Einstellung des Hörgeräts, genauer der Betriebsparameter, erfolgen kann. Eine EEG-Messung ist jedoch apparativ sehr aufwendig. Insgesamt ist ein EEG daher nicht alltagstauglich.

In der US 2012/0245655 A1 ist im Gegensatz zu einer reinen Messung der Hirnströme eine Messung eines Muskelreflexes beschrieben, nämlich eines Reflexes des hinteren Ohrmuskels (Englisch: post-auricular muscle), weshalb der Reflex auch als PAMR, kurz für post-auricular muscle reflex, bezeichnet wird. Dieser Reflex wird durch einen akustischen Reiz ausgelöst, vornehmlich ein lautes plötzliches Geräusch, wie z.B. ein Knall. Durch Messung ebenjenes Reflexes Ist es dann möglich, eine reflexhafte Reaktion einer Person auf einen entsprechenden akustischen Reiz zu erkennen. Der Reflex des hinteren Ohrmuskels wird mittels eines Elektrodenarrays mit einer Vielzahl an Elektroden gemessen. Bei einem Signalanstieg oder bei Erreichen eines Schwellwerts wird der Reflex als solcher erkannt. Basierend auf dieser Erkennung kann dann eine Einstellung von Betriebscharakteristika des Hörgeräts erfolgen.

In der US 2010/0160714 A1 wird ein Hörgerät beschrieben, welches Gehirnwellensignale misst, um davon abhängig eine Hörgerätekomponente zu steuern. Die Gehirnwellensignale können EEG-, MEG-, EMG-, MRI- oder NMR-Signale sein.

Vor diesem Hintergrund ist es eine Aufgabe der Erfindung, ein verbessertes Verfahren zum Betrieb eines Hörgeräts anzugeben sowie ein entsprechendes Hörgerät. Dabei soll eine Anzahl an Betriebsparametern des Hörgeräts möglichst zuverlässig anhand der Bedürfnisse eines Anwenders in einer konkreten Situation erfolgen. Das Verfahren und das Hörgerät sollen zudem möglichst alltagstauglich sein.

Die Aufgabe wird erfindungsgemäß gelöst durch ein Verfahren mit den Merkmalen gemäß Anspruch 1 sowie durch ein Hörgerät mit den Merkmalen gemäß Anspruch 15. Vorteilhafte Ausgestaltungen, Weiterbildungen und Varianten sind Gegenstand der Unteransprüche. Dabei gelten die Ausführungen im Zusammenhang mit dem Verfahren sinngemäß auch für das Hörgerät und umgekehrt.

Das Verfahren dient zum Betrieb eines Hörgeräts. Das Hörgerät ist zur Anwendung durch einen Anwender ausgebildet. Bei dem Verfahren wird eine Elektromyographie durchgeführt, bei welcher mittels eines Elektrodenarrays eine Muskelaktivität eines Ohrmuskels des Anwenders gemessen wird. Die Muskelaktivität ist insbesondere das Resultat einer Orientierungsaktivität des Ohrmuskels, zur Ausrichtung des zugehörigen Ohrs des Anwenders, wobei diese Orientierungsaktivität beim Menschen jedoch evolutionsbiologisch bedingt stark unterentwickelt und daher lediglich rudimentär ausgebildet ist. Die Muskelaktivität wird fortlaufend gemessen, zur Erfassung eines komplexen Aktivitätsprofils des Ohrmuskels, in welchem eine Intention des Anwenders kodiert ist, d.h. in der Muskelaktivität ist eine Intention des Anwenders kodiert. Die Intention wird speziell auch als Hörintention bezeichnet. Das Elektrodenarray erzeugt ein Sensorsignal, welches mittels eines Klassifikators einer Klassifikation unterzogen wird. Bei der Klassifikation wird die Muskelaktivität dekodiert und die zugrunde liegende Intention bestimmt, indem untersucht wird, ob das Sensorsignal einen vorbekannten Merkmalsvektor aufweist, welcher zu einer vorbekannten Klasse gehört.. Dem vorbekannten Merkmalsvektor, d.h. der vorbekannten Klasse, ist ein Betriebsmodus des Hörgeräts zugeordnet, welcher eingestellt wird, wenn das Sensorsignal den vorbekannten Merkmalsvektor aufweist, d.h. wenn die Intention erkannt wird. Es wird also die Intention des Anwenders aus der Muskelaktivität der Ohrmuskulatur ermittelt und anhand dieser Intention wird das Hörgerät eingestellt, d.h. es wird ein bestimmter Betriebsmodus eingestellt, welcher der Intention zugeordnet ist, um in derjenigen Situation, welche Anlass der Intention ist, einen optimalen Betrieb des Hörgeräts zu gewährleisten.

Der Betrieb des Hörgeräts ist zu einem gegebenen Zeitpunkt durch bestimmte Werte für eine Anzahl an Betriebsparametern definiert. Diese Betriebsparameter und deren Werte bilden einen Betriebsmodus des Hörgeräts. Ein Betriebsparameter ist z.B. ein Verstärkungsfaktor, mit welchem eingehende Schallsignale vor einer Ausgabe an den Anwender verstärkt werden. Ein jeweiliger Betriebsmodus dient dazu, das Verhalten des Hörgeräts in einer bestimmten Situation, welche dem Betriebsmodus zugeordnet ist, optimal auf die Bedürfnisse des Anwenders in der bestimmten Situation anzupassen. Unter einem "Einstellen eines Betriebsmodus" wird verstanden, dass ein oder mehrere Betriebsparameter in vorbestimmter Weise, z.B. auf bestimmte Werte, eingestellt werden. Grundsätzlich können auch mehrere Betriebsmodi gleichzeitig eingestellt sein, wobei sich dann die Werte der Betriebsparameter z.B. als Mittelwert der Werte unterschiedlicher Betriebsmodi ergeben.

Bei dem vorliegenden Verfahren wird eine Elektromyographie, kurz EMG, durchgeführt, bei welcher mittels eines Elektrodenarrays eine Muskelaktivität eines Ohrmuskels des Anwenders gemessen wird. Diese Muskelaktivität ist das Resultat eines Nervensignals, welches vom Gehirn an den Ohrmuskel gesendet wird. Entsprechend wird mittels des Elektrodenarrays ein bioelektrisches Signal gemessen, nämlich ein elektrisches Potential des Ohrmuskels. Das bioelektrische Signal geht vom Ohrmuskel aus, im Gegensatz zu einem EEG wird also nicht das Nervensignal des Gehirns gemessen, sondern ein Signal am Ohrmuskel. Ein wesentlicher Vorteil der EMG-Messung besteht insbesondere darin, dass das zu messende Signal deutlich stärker und isolierter ist als bei einer EEG-Messung. Im Rahmen einer EEG-Messung wird das Nervensignal gemessen, welches vom Gehirn gesendet wird, dieses Nervensignal wird jedoch am Ohrmuskel über weitere Motoreinheiten verstärkt, sodass für die EMG-Messung ein stärkeres Signal zur Verfügung steht. Der im Vergleich zum Gehirn weiter außen am Kopf angeordnete Ohrmuskel wird auch nicht durch die zahlreichen anderen Signale und Reaktionen anderer Nerven und Muskel beeinflusst, wohingegen ein EEG notwendigerweise eine Überlagerung einer Vielzahl an Nervensignalen misst. Mit anderen Worten: das EMG-Signal ist bereits auf natürliche Weise gefiltert und verstärkt. Bei einem EMG ist also im Vergleich zu einem EEG die Signalqualität deutlich besser und es steht mehr Nutzsignal zur Analyse zur Verfügung. Dadurch entfällt die Notwendigkeit eines entsprechenden apparativen Aufwands zur Signalaufbereitung, wodurch das Hörgerät deutlich kompakter ausfällt und überhaupt erst alltagstauglich wird. Weiterhin weist das Sensorsignal der EMG-Messung gegenüber einer EEG-Messung höherfrequente Frequenzkomponenten auf, sodass eine EMG-Messung im Vergleich schneller durchführbar ist und sich eine kürzere Messzeit ergibt.

Das Elektrodenarray dient zur Erstellung des Sensorsignals. Hierzu weist das Elektrodenarray eine Anzahl von einzelnen Elektroden oder auch Polen auf, welche an unterschiedlichen Stellen des Kopfes des Anwenders angeordnet sind. Eine einzelne Elektrode ist auch ein einzelner elektrischer Kontakt. Um nun die Muskelaktivität zu messen, wird die Potentialdifferenz zwischen zwei unterschiedlichen Elektroden gemessen. Die Potentialdifferenz ergibt dann das Sensorsignal und entspricht diesem insbesondere. Das Elektrodenarray ist also multipolar ausgebildet, um das Sensorsignal als Potentialdifferenz zwischen zwei einzelnen Kontakten an unterschiedlichen Stellen des Kopfes des Anwenders zu messen.

Im Rahmen dieser Anmeldung wird unter "Ohrmuskel" einer der Muskeln der Ohrmuskulatur des Menschen verstanden. Die Ohrmuskulatur besteht aus den Muskeln auricularis anterior, auricularis posterior, auricularis superior, helicis major, helicis minor, transversus auriculae, obliquus auriculae, tragicus und antitragicus. Die vorgenannte Liste ist abschließend. Vorzugsweise ist der Ohrmuskel bei dem Verfahren einer der drei extrinsischen Ohrmuskeln, d.h. auricularis anterior, posterior und superior, ganz besonders bevorzugt ist der hintere Ohrmuskel, d.h. des musculus auricularis posterior, da dieser im Tragebereich eines hinter dem Ohr getragenen Hörgeräts liegt.

Die Ohrmuskeln erfüllen beim Menschen zumindest heutzutage keine relevante Funktion mehr, werden aber dennoch in manchen Situationen und vor allem auf akustische Reize hin vom Gehirn angesteuert. Eine tatsächlich willentliche Anregung der Ohrmuskeln ist den meisten Menschen nicht möglich, allerdings erfolgt bei den allermeisten Menschen eine Anregung durch das Gehirn insofern willensgesteuert, als dass die Ohrmuskulatur bei gewollten, d.h. intendierten Aktionen eines Menschen in bestimmter Weise mit angeregt wird. Die Muskelaktivität, welche mittels des Elektrodenarrays erfasst wird, ist dann eine Orientierungsaktivität, d.h. eine Aktivität, welche zum Ziel hat, das Ohr auszurichten, diese Anstrengung bleibt im Ergebnis aber fruchtlos, da eine solche Ausrichtung der Ohren beim Menschen tatsächlich nicht möglich ist. Die Fähigkeit, die Ohren mithilfe der Ohrmuskulatur willentlich auszurichten, ist jedoch bei einigen Tieren vorhanden. Das Phänomen ist ausführlich beschrieben in Hackley et al. "Combined Use of Microreflexes and Event-Related Brain Potentials as Measures of Auditory Selective Attention", November 1987, Psychophysiology Vol.24, No.6 S.632ff und Hackley "Evidence for a vestigal pinna-orienting system in humans", 2015, Psychophysiology, Vol.52, S.1263ff. Im Rahmen der vorliegenden Erfindung wird nun die Erkenntnis ausgenutzt, dass beim Menschen eine Muskelaktivität bei einer entsprechenden Intention dennoch erfolgt.

Die Messung der Muskelaktivität, d.h. allgemein der Aktivität der Ohrmuskulatur des Anwenders oder auch das Verhalten eines oder mehrerer der Ohrmuskeln der Ohrmuskulatur, stellt ein Kernkonzept der Erfindung dar. Diesem Ansatz liegt die Erkenntnis zugrunde, dass die Muskelaktivität der Ohrmuskeln durch das Gehirn des Anwenders veranlasst ist, dass also bei Vorliegen einer bestimmten Situation mit bestimmten Reizen bereits vom Gehirn und somit vom Anwender entschieden wurde, dass ein potentiell relevanter Reiz vorliegt. Bei Sensoren, welche lediglich externe Effekte wie Lautstärke, Beschleunigung oder dergleichen messen ist eine Vorentscheidung durch das Gehirn also durch den Anwender selbst nicht möglich. Mit anderen Worten: mit der Aktivität der Ohrmuskeln korreliert eine Intention, genauer eine Hörintention des Anwenders. Besonders bei einer bewussten Hörintention, bei welcher sich der Anwender anstrengt, auf ein bestimmtes Schallsignal zu hören, z.B. in einer bestimmten Richtung, wird die Ohrmuskulatur aktiv.

In der Muskelaktivität ist also eine Intention des Anwenders kodiert, denn die Muskelaktivität ist nicht ausschließlich reflexartig, sondern ergibt sich auch bei einer willentlichen Anstrengung, wie Hackley (2015, a.a.O.) beschreibt. Die Muskelaktivität ist daher auch ein Ausdruck des Willens des Anwenders in einer bestimmten Richtung oder auf eine bestimmte Art oder auf einen bestimmten Reiz hin zu hören. Aufgrund dieser Hörintention veranlasst das Gehirn eine Anregung der Ohrmuskulatur. Das Gehirn und der Ohrmuskel wirken somit nach Art eines Klassifikators, d.h. die aktuelle Situation wurde vom Gehirn derart klassifiziert, dass als Antwort hierauf eine Muskelaktivität zur Orientierung der Ohrmuskeln erfolgen soll. Bei dem vorliegenden Verfahren wird also die willentliche Hörintention des Anwenders aus der Muskelaktivität der Ohrmuskulatur dekodiert und dann genutzt, um das Hörgerät einzustellen.

Eine Einstellung des Hörgeräts wird also nicht oder zumindest nicht alleinig durch die Umgebung des Anwenders direkt veranlasst und dabei möglicherweise unabhängig von der Intention des Anwenders. Vielmehr wird ausgenutzt, dass die Muskelaktivität auch eine bestimmte Relevanz der aktuellen Situation für das Hören indiziert, sodass ein Einstellen in Kenntnis der Intention des Anwenders durchgeführt wird, d.h. vorteilhafterweise genau dann, wenn dies vom Anwender gewollt ist. Auf Basis der Aktivität der Ohrmuskulatur wird also die Intention des Anwenders mit berücksichtigt und daraufhin eine Einstellung des Hörgeräts vorgenommen, welche im Ergebnis deutlich bedarfsgerechter und weniger fehlerbehaftet ist. Fehleinstellungen werden vorteilhaft vermieden.

Ein wesentlicher Aspekt bei der Messung der Muskelaktivität ist, dass nicht lediglich ein Reflex gemessen wird. Vorliegend wurde nämlich erkannt, dass ein Reflex auf einen knallartigen akustischen Reiz lediglich ein einzelnes Element einer an sich deutlich komplexeren Muskelaktivität ist. Die Ohrmuskulatur spricht gerade nicht nur reflexartig und zeitlich betrachtet punktuell auf bestimmte Reize und Situationen an, sondern setzt sich je nach Situation auch über einen längeren Zeitraum von z.B. mehreren Sekunden fort und ist gerade nicht lediglich reflexartig, sondern wie oben beschrieben auch Ausdruck einer Intention des Anwenders. Typischerweise ergibt sich ein Reflex des Ohrmuskels bei Wahrnehmung eines plötzlichen akustischen Reizes wie z.B. eines Knalls und innerhalb eines Zeitraums von bis zu 25ms nachdem das Schallsignal das Ohr erreicht hat. Im Rahmen des Reflexes ist eine besonders starke Änderung des Potentials am Ohrmuskel messbar. Dies wird in der eingangs erwähnten US 2012/0245655 A1 ausgenutzt.

Bei dem Verfahren der vorliegenden Anmeldung wird jedoch nicht eine bloße reflexartige Muskelreaktion gemessen, sondern darüber hinaus die Muskelaktivität, also auch das Antwortverhalten des Ohrmuskels jenseits eines kurzzeitigen Reflexes. Die Ohrmuskulatur zeigt also abseits eines einfachen Reflexes auch noch ein deutlich komplexeres Aktivitätsprofil, welches bei dem vorliegenden Verfahren gemessen wird, indem die Muskelaktivität fortlaufend gemessen wird. Beispielsweise wurde vorliegend erkannt, dass die Muskelaktivität nicht lediglich einzelne isolierte Signalstöße aufweist, wie beim Reflex, sondern ganze Folgen von zusammengehörigen Signalstößen. Im Gegensatz zu einer Reflexmessung wird daher vorliegend die gesamte Muskelaktivität des Ohrmuskels nicht auf einen bestimmten Schwellwert getriggert überwacht, sondern über einen längeren Zeitraum, d.h. es wird eine Langzeitmessung durchgeführt, um das gesamte und komplexe Aktivitätsprofil des Ohrmuskels zu erfassen. Unter "fortlaufend" wird daher insbesondere verstanden, dass die Muskelaktivität nicht durch das Sensorsignal selbst getriggert erfolgt, sondern über einen bestimmten Messzeitraum, welcher insbesondere unabhängig vom Sensorsignal ist. Die Langzeitmessung dient dabei nicht der Mittelwertbildung über mehrere wiederholt auftauchende Signale, sondern vielmehr der Erfassung komplexer Aktivitätsprofile, welche bei einer lediglich getriggerten oder kurzen Messung nicht mit erfasst würden. Dadurch wird abseits von eventuellen Reflexen auch eine möglicherweise andauernde Muskelaktivität mit erfasst, gemessen und ausgewertet. Vorteilhafterweise bleibt dann eine Schallquelle, auf welche der Anwender nicht hören will und bezüglich derer er keine Hörintention äußert, durch das Hörgerät unberücksichtigt und führt nicht zu einer anderen Einstellung, obwohl das Hörgerät das Vorhandensein dieser Schallquelle möglicherweise auf andere Weise bereits ermittelt hat, z.B. durch Analyse eines Mikrofonsignals.

Durch eine Messung des Verhaltens eines Ohrmuskels über einen längeren Zeitraum wird also ein komplexer Signalverlauf des Sensorsignals produziert, welcher nunmehr nach Mustern abgesucht wird, um das Sensorsignal zu klassifizieren und die Intention des Anwenders zu dekodieren. Die gesuchten Muster sind dabei insbesondere deutlich komplizierter und somit situationsspezifischer als bei einem einfachen Reflex. Ein einzelner Reflex wie oben beschrieben ist dabei jedoch nicht zwingend bedeutungslos, vielmehr werden durch die Messung über einen längeren Zeitraum auch mehrere Reflexe gemeinsam erfasst, welche dann z.B. als Reflexgruppe ausgewertet werden, welche möglicherweise eine bestimmte Intention des Anwenders beschreibt. Die Langzeitmessung ist dabei vorteilhaft nicht auf die Messung von Reflexen beschränkt, sondern es wird vielmehr das Langzeitverhalten des Ohrmuskels gemessen. Vorzugsweise wird sogar gänzlich auf eine Reflexmessung verzichtet, denn in dieser ist - da es sich ja lediglich um einen Reflex handelt - üblicherweise keine Intention des Anwenders kodiert.

Das Sensorsignal ist insbesondere eine Spannung, welche sich in Abhängigkeit der Muskelaktivität, d.h. genauer gesagt des Potentials des Muskels ändert und z.B. proportional hierzu ist. Das Sensorsignal wird mittels eines Klassifikators klassifiziert, d.h. einer Klassifikation unterzogen und mit vorbekannten Merkmalsvektoren verglichen, um das Sensorsignal einer Klasse zuzordnen. Zuvor wird das Sensorsignal jedoch zweckmäßigerweise in einem Vorfilter vorgefiltert, wobei insbesondere Rauschartefakte entfernt werden. Ein Merkmalsvektor weist allgemein eine Anzahl von üblicherweise mehreren Merkmalen auf. Diese Merkmale sind z.B. bestimmte Amplituden, Schwellwerte, bestimmte Verläufe, Signalfolgen oder das bloße Vorhandensein bestimmter Signale oder Signalanteile im gesamten Sensorsignal. Das Sensorsignal wird also hinsichtlich solcher Merkmale untersucht. Liegen die Merkmale eines Merkmalsvektors hinreichend deutlich vor, d.h. innerhalb eines Toleranzbereichs, wird der Merkmalsvektor in dem Sensorsignal erkannt. Jeder vorbekannte Merkmalsvektor entspricht dann einer bestimmten Muskelaktivität. Dem liegt die Überlegung zugrunde, dass jede Intention des Anwenders eine bestimmte Signatur innerhalb der Muskelaktivität erzeugt, welche durch eine Anzahl an Merkmalen und dadurch durch einen Merkmalsvektor charakterisiert ist und welche damit klassifizierbar ist. Jeder vorbekannte Merkmalsvektor entspricht also einer Intention des Anwenders, sodass durch eine Klassifikation des Sensorsignals und eine Ermittlung der darin enthaltenen Merkmalsvektoren die Intentionen des Anwenders dekodiert werden und als Steuerkommandos für das Hörgerät verwendet werden. Durch die fortlaufende Messung der insbesondere anhaltenden Muskelaktivität werden auch komplexe und zeitstabile Merkmalsvektoren erfasst, sodass eine besonders differenzierte Erkennung der Intention des Anwenders möglich ist und auch erfolgt. Eine Auswertung des Sensorsignals erfolgt also nicht beschränkt auf kleine Zeiteinheiten von insbesondere weniger als 100ms, sondern auf einer größeren Zeitskala, sodass auch eine komplexe und andauernde Aktivität zuverlässig erfasst wird.

Wie bereits angedeutet, ist im Raum der Merkmalsvektoren eine Anzahl von Klassen ausgebildet, welchen die Merkmalsvektoren zugeordnet sind, d.h. die vorbekannten Merkmalsvektoren sind jeweils einer Klasse zugeordnet. Der Merkmalsvektor, welcher in dem Sensorsignal ermittelt wird, also der Merkmalsvektor des Sensorsignals, wird dann je nach Ausgestaltung, d.h. je nach konkreten Merkmalen, einer der Klassen zugeordnet. Eine Klasse ergibt sich also als Menge ähnlicher Merkmalsvektoren. Eine jeweilige Klasse deckt dabei einen Teilraum des gesamten Raums der Merkmalsvektoren ab, ist also eine Teilmenge, welche all jene Merkmalsvektoren enthält, welche demselben Betriebsmodus zugeordnet sind, welche also hinreichen ähnlich sind. Im Folgenden wird jedoch zuweilen vereinfachend der Begriff "vorbekannter Merkmalsvektor" anstelle des Begriffs Klasse verwendet.

Als Merkmal wird vorzugsweise der Organisationsgrad oder die Energie des Sensorsignals verwendet oder beides. Ein bevorzugtes Maß für den Organisationsgrad ist die Entropie des Sensorsignals, wobei die Entropie definiert ist als die Änderung des Sensorsignals im Verlauf der Zeit, insbesondere innerhalb des Messzeitraums. Hierbei wird beispielsweise die Änderung der Amplitude untersucht. Die Energie ist definiert als das Integral über das Sensorsignal im Verlauf der Zeit, insbesondere innerhalb des Zeitraums der Messung. In einer Variante ist zur Bestimmung der Energie das Sensorsignal mit einem Exponenten gewichtet, d.h. es wird eine Norm höherer Ordnung verwendet, z.B. eine quadratische Norm. Eine niedrige Norm führt zu einer verringerten Varianz innerhalb einer Klasse (Englisch: in class variance), d.h. die Anzahl von Merkmalsvektoren einer einzelnen Klasse variieren weniger und sind sich somit ähnlicher. Demgegenüber führt eine hohe Norm zu einer verringerten Varianz zwischen verschiedenen Klassen (Englisch: inter class variance), die Ähnlichkeit der Merkmalsvektoren, welche einer Klasse zugeordnet werden ist also verringert, allerdings werden Unterschiede der Merkmalsvektoren zueinander stärker betont, sodass sich feiner aufgelöste Klassen bilden lassen. Bevorzugt ist vorliegend eine möglichst niedrige Norm, insbesondere eine L¹-Norm.

Besonders bevorzugt werden mehrere der vorgenannten Merkmale dadurch gebildet, dass die entsprechende Untersuchung in verschiedenen Frequenzbändern erfolgt. Das Sensorsignal innerhalb eines bestimmten Messzeitraums wird also im Frequenzraum betrachtet und hier in mehrere Frequenzbänder unterteilt, welche jeweils separat auf deren Entropie, Energie oder beides untersucht werden. In letzterem Fall ergeben sich also zweimal so viele Merkmale wie Frequenzbänder. Die vorbekannten Merkmalsvektoren sind dann charakterisiert durch bestimmte Werte, Wertebereiche, Schwellwerte, Grenzwerte, Änderungsmuster, Verläufe oder Ähnliches für eines oder mehrere der Merkmale.

Das Antwortverhalten des Ohrmuskels wird insbesondere kontinuierlich überwacht und dabei wird ein entsprechend kontinuierliches Sensorsignal erzeugt, dessen Verlauf eine deutlich umfangreichere Analyse ermöglicht als bei einer Kurzzeitmessung oder einer Mittelwertbildung über mehrere kurze Zeitfenster. Unter "längere Zeit" wird vorzugsweise ein Zeitraum, d.h. Messzeitraum verstanden, welcher länger ist als ein einzelner Reflex des Ohrmuskels und welcher insbesondere länger als 25ms ist, vorzugsweise länger als 100ms und besonders bevorzugt länger als 1s. Der vorbekannte Merkmalsvektor wird hierbei über den gesamten Messzeitraum in dem Sensorsignal gesucht. Dadurch wird sichergestellt, dass auch Merkmalsvektoren erkannt werden, welche eine längere und andauernde Muskelaktivität abbilden. Dem liegt die Überlegung zugrunde, dass ein Merkmalsvektor nicht zwingend lediglich ein einzelnes Merkmal enthält oder solche Merkmale, welche zeitgleich vorliegen, sondern dass sich im Rahmen des komplexen Aktivitätsprofils des Ohrmuskels auch zeitlich versetzte Merkmale durch eine einzelne Intention veranlasst sind und sich dieser dann zuordnen lassen oder umgekehrt, dass die Intention durch zeitlich auseinanderliegende Merkmale definiert ist. Durch die Untersuchung des gesamten, längeren Messzeitraums werden also auch solche Merkmalsvektoren erkannt, welche eine bestimmte Dynamik der Muskelaktivität beschreiben und eben nicht lediglich kurze Reflexe. Eine obere Grenze ist dabei grundsätzlich nicht gegeben, zweckmäßigerweise ist der Messzeitraum aber nicht länger als 10s, da einerseits Reaktionen, welche weiter auseinanderliegen, üblicherweise nicht zusammengehören und daher üblicherweise keinen Mehrwert hinsichtlich der Auswertung aufweisen. Andererseits würde durch einen zu langen Messzeitraum auch die Antwortgeschwindigkeit des Hörgeräts zu stark verlangsamt, das Hörgerät würde also die Intention des Anwenders nur stark zeitverzögert erkennen und entsprechend zeitverzögert reagieren. Besonders zweckmäßig ist ein sogenannter rollender Zeitraum, welcher über die Zeit mitwandert.

Die Überwachung der Muskelaktivität der Ohrmuskulatur bietet eine Vielzahl an Anwendungsmöglichkeiten. Die beschriebene Klassifikation ermöglicht insbesondere in Kombination mit den weiter unten beschriebenen Konzepten zum Erlernen neuer Merkmalsvektoren eine Reaktion auf jegliche Aktivität des Ohrmuskels.

Da die Ohrmuskulatur im Grunde bei jeglicher Aktivierung eine Orientierungsaktivität der Ohren anstrebt, ist eine Überwachung der Muskelaktivität besonders geeignet zum Einstellen einer Richtungscharakteristik, kurz Richtcharakteristik, des Hörgeräts, also zur Anpassung eines Richtungshörens. Beim Richtungshören gibt das Hörgerät die aufgenommenen Schallsignale der Umgebung derart an den Anwender aus, dass Schallsignale aus einer bestimmten Richtung, nämlich einer Hörrichtung, gegenüber den übrigen Schallsignalen verstärkt werden. Dadurch werden Schallsignale aus der Hörrichtung bevorzugt wiedergegeben. In einer vorteilhaften Ausgestaltung ist das Hörgerät dann zum Richtungshören ausgebildet und anhand der Muskelaktivität wird eine Richtcharakteristik des Hörgeräts eingestellt, wodurch eine Schallquelle in einer bestimmten Richtung hervorgehoben wird. Die Richtcharakteristik ist insbesondere eine Richtkeule, welche durch eine Breite und einen Winkel definiert ist. Schallquellen innerhalb der Richtkeule werden vom Hörgerät mehr verstärkt als Schallquellen außerhalb der Richtkeule. Der Winkel gibt an, in welcher Richtung bezüglich des Anwenders die Richtkeule ausgerichtet ist. Die Breite gibt an, welchen Winkelbereich die Richtkeule abdeckt. Der Winkel und die Breite sind dann jeweils Betriebsparameter, welche gemeinsam einen Betriebsmodus "Richtungshören in einer bestimmten Hörrichtung" bilden.

Beim Einstellen der Richtcharakteristik in Abhängigkeit der Muskelaktivität wird ausgenutzt, dass die Muskelaktivität evolutionsbiologisch eine Ausrichtung der Ohren bewirken soll, insofern ist in der Muskelaktivität der Ohrmuskeln eine Intention zum Hören in einer bestimmten Richtung kodiert. Eine Ausrichtung der Ohren findet wie bereits gesagt tatsächlich nicht statt, die Steuersignale an die Ohrmuskeln sind jedoch vorhanden und weisen eine entsprechende Signatur auf, welche ein entsprechendes Aktivitätsprofil ergibt und welche üblicherweise anwenderspezifisch ist. Durch Kenntnis dieser Signatur, d.h. des zugehörigen Merkmalsvektors, wird dann die Intention eines Richtungshörens erkannt. Dabei wird in einer Variante nicht lediglich erkannt, dass ein Richtungshören gewünscht ist, sondern es wird vielmehr auch die gewollte Hörrichtung erkannt. Entsprechendes gilt für die Breite der Richtkeule.

Die Signatur beim Richtungshören besteht insbesondere aus einer Folge von Signalspitzen, d.h. das Sensorsignal weist in bestimmten, definierten und insbesondere gleichbleibenden Abständen eine erhöhte Amplitude auf. Beim Richtungshören wird also vorzugsweise in dem Sensorsignal eine Folge von Signalspitzen gesucht. Die Abstände sind beim Richtungshören insbesondere derart, dass die Signalspitzen im Frequenzraum einen hochfrequenten Signalanteil ergeben, insbesondere im Bereich zwischen 500Hz und 1000Hz.

In einer besonders bevorzugten Ausgestaltung ist das Hörgerät ein binaurales Hörgerät und weist zwei Einzelgeräte auf, zum Tragen auf unterschiedlichen Seiten des Kopfes des Anwenders. Die aktuelle Situation wird dann erkannt, indem auf jeder Seite mittels eines jeweiligen Elektrodenarrays eine Muskelaktivität eines dortigen Ohrmuskels gemessen wird. In dieser Konfiguration stehen also vorteilhaft zwei Sensorsignale zur Verfügung und somit insgesamt mehr Informationen, d.h. Merkmale zur Klassifizierung der Intention des Anwenders und zur verbesserten Auswahl eines geeigneten Betriebsmodus. Grundsätzlich geeignet ist aber auch ein monoaurales Hörgerät, mit lediglich einem Einzelgerät, zur Versorgung lediglich eines Ohrs des Anwenders.

Vorzugsweise werden die Muskelaktivitäten auf den beiden Seiten zunächst getrennt ausgewertet, indem die Sensorsignale unabhängig voneinander klassifiziert werden, und anschließend wird bei einem übereinstimmenden Ergebnis das Hörgerät eingestellt. Insbesondere wird lediglich bei einem übereinstimmenden Ergebnis das Hörgerät eingestellt. Es erfolgt also eine komparative Entscheidungsfindung, bei welcher das Ergebnis vom Vergleich zweier unabhängig ausgewerteter Sensorsignale abhängt. Die beiden Klassifikatoren liefern jeweils unabhängig voneinander ein Klassifikationsergebnis und die beiden Klassifikationsergebnisse werden miteinander verglichen. Stimmen die beiden Klassifikationsergebnisse überein, wird das Hörgerät entsprechend eingestellt. Mit anderen Worten: der Betriebsmodus wird zunächst redundant ermittelt, indem die beiden Sensorsignale separat klassifiziert werden, sodass zwei Betriebsmodi ermittelt werden. Falls die beiden Betriebsmodi gleich sind, also für beide Seiten dieselbe Intention erkannt wurde, wird ein entsprechender Betriebsmodus eingestellt. Andernfalls erfolgt insbesondere keine Einstellung des Hörgeräts in Abhängigkeit der Muskelaktivität. Hierdurch werden Fehleinschätzungen verhindert. In einer Variante wird ein Mischbetrieb eingestellt, welcher sich sozusagen als Kompromiss als eine Mischung der beiden ermittelten Betriebsmodi ergibt.

Zusätzlich oder alternativ wird die Muskelaktivitäten auf den beiden Seiten zunächst getrennt ausgewertet, indem die Sensorsignale unabhängig voneinander klassifiziert werden, und bei unterschiedlichen Ergebnissen werden die Einzelgeräte synchronisiert, indem das eine der Einzelgeräte entgegen des eigenen Ergebnisses und anhand des Ergebnisses des anderen Einzelgeräts eingestellt wird. Bei unterschiedlichen Ergebnissen, genauer Klassifikationsergebnissen werden auch unterschiedliche optimale Betriebsmodi bestimmt. Anstatt wie oben beschrieben nur bei übereinstimmendem Ergebnis eine Einstellung vorzunehmen, wird also nun sozusagen versucht eine Einigung zwischen den beiden Ergebnissen der Einzelgeräte zu erzielen, um auch bei divergierendem Ergebnis der Auswertung der Sensorsignale einen möglichst optimalen Betriebsmodus einzustellen. Beispielsweise wird bei unterschiedlichen Ergebnissen zusätzlich ein Zusatzsensor ausgewertet, um zu entscheiden, welches der beiden Ergebnisse verwendet wird und welcher Betriebsmodus dann eingestellt wird. Alternativ oder zusätzlich ist eines der beiden Einzelgeräte voreingestellt, z.B. in einem Trainingsverfahren trainiert, und trainiert dann bei unterschiedlichem Ergebnis das andere Einzelgerät, indem der vom voreingestellten Einzelgerät ermittelte Betriebsmodus eingestellt wird.

Das oben beschriebene binaurale Hörgerät eignet sich besonders zum weiter oben beschriebenen Einstellen einer Richtcharakteristik im Rahmen eines Richtungshörens, d.h. eines gerichteten Hörens.

In einer geeigneten Ausgestaltung zum Richtungshören wird eine gewollte Hörrichtung, d.h. eine Intention, insbesondere qualitativ vorzugsweise dadurch ermittelt, dass untersucht wird, welches der beiden Sensorsignale einen höheren Organisationsgrad aufweist oder eine höhere Energie. Auf dieser Seite liegt dann die Schallquelle. Die Richtcharakteristik wird dann in Richtung der Hörrichtung eingestellt. Dem liegt die Überlegung zugrunde, dass bei einer seitlichen Schallquelle, d.h. einer nicht frontal und nicht mittig positionierten Schallquelle, lediglich das Ohr auf der entsprechenden Seite ausgerichtet werden müsste, das also auf dieser Seite eine besondere Anstrengung der Ohrmuskulatur zu messen ist, während die andere Seite weitgehend ruhig bleibt, sodass das Sensorsignal einen geringeren Organisationsgrad aufweist. Diese qualitative Ermittlung der Hörintention ist insofern vorteilhaft auch wenig anwenderspezifisch und dadurch generalisierbar. Unter "höherem Organisationsgrad" wird allgemein insbesondere verstanden, dass in dem entsprechenden Sensorsignal mehr Merkmale oder überhaupt irgendwelche Merkmale gefunden werden und speziell insbesondere, dass auf einer Seite die Entropie in einem oder mehreren oder allen Frequenzbändern größer ist als auf der anderen Seite. Unter "höherer Energie" wird dann verstanden, dass die Energie in einem, mehreren oder allen Frequenzbändern auf einer Seite höher ist, wobei die Energie insbesondere wie oben bereits beschrieben bestimmt wird.

Alternativ oder zusätzlich zu der beschriebenen qualitativen Untersuchung wird die gewollte Hörrichtung quantitativ ermittelt, d.h. insbesondere dass die Richtung oder die Breite der Richtkeule oder beides ermittelt werden. Auch hierzu wird die Muskelaktivität entsprechend ausgewertet. Dabei werden die Sensorsignale auf beiden Seiten entweder redundant ausgewertet, wie oben beschrieben, oder gemeinsam oder beides in Kombination und z.B. nacheinander. Bei der gemeinsamen Auswertung, ist ein Merkmalsvektor aus Merkmalen beider Seiten zusammengesetzt, d.h. die Merkmale auf beiden Seiten werden kombiniert verwendet, um einen beide Seiten überspannenden Merkmalsvektor in dem Sensorsignal zu ermitteln und diesen mit entsprechenden vorbekannten Merkmalsvektoren zu vergleichen. Auf diese Weise wird die Merkmalsanzahl vorteilhaft erhöht und die Ermittlung des geeigneten Betriebsmodus noch spezifischer.

In der Orientierungsaktivität der Ohrmuskeln ist jedoch nicht lediglich eine Hörintention kodiert, vielmehr kann die Ausrichtung der Ohrmuskeln auch als Teil einer Gesichtsmimik des Anwenders aufgefasst werden. Insbesondere hat insgesamt eine Änderung der Gesichtsmimik oder auch eine Bewegung einzelner Gesichtspartien eine Auswirkung auf die Ohrmuskulatur. Von besonderem Interesse ist vorliegend eine Muskelaktivität des Ohrmuskels beim Sprechen und beim Formen eines Gesichtsausdrucks.

Die Muskelaktivität beim Sprechen wird in einer bevorzugten Ausgestaltung zur Eigenstimmendetektion verwendet. Anhand der Muskelaktivität wird dann eine Eigenstimmensituation erkannt, bei welcher der Anwender selbst spricht. Der Betriebsmodus, welcher eingestellt wird, ist eine Eigenstimmenreduktion, bei welcher ein Eigenstimmenanteil in einem Ausgangssignal des Hörgeräts reduziert, insbesondere herausgefiltert wird. Bei einem binauralen Hörgerät erfolgt dies vorzugsweise mittels einer binauralen Verrechnung, d.h. durch geeignete Kombination von Mikrofonsignalen von beiden Seiten des Kopfes des Anwenders.

In einer zweckmäßigen Weiterbildung wird nicht nur eine Eigenstimmensituation an sich erkannt, sondern es wird auch erkannt, was genau gesprochen wird, es erfolgt also eine Spracherkennung. Der Eigenstimmenanteil wird dann besonders gezielt und effektiv reduziert oder sogar gänzlich eliminiert. In einer ersten Variante erfolgt die Spracherkennung mittels eines Zusatzsensors und die Eigenstimmenerkennung anhand der Muskelaktivität dient als zusätzliche Information, welche die Spracherkennung unterstützt. Dem liegt die Überlegung zugrunde, dass ein Zusatzsensor, z.B. ein Mikrofon, unter Umständen nicht optimal zwischen eigener und fremder Stimme unterscheiden kann. Die Eigenstimmenerkennung als zusätzliche Information unterstützt dann die Spracherkennung dahingehend, dass sauberer zwischen fremder und eigener Stimme unterschieden wird. Die Spracherkennung wird beispielsweise mittels eines Klassifikators ausgeführt, welcher die Mikrofonsignale untersucht, sodass die zusätzliche Berücksichtigung der Muskelaktivität weitere Merkmale liefert und auf diese Weise den Klassifikator unterstützt. In einer zweiten Variante, welche auch zusätzlich verwendet werden kann, erfolgt eine Spracherkennung alleinig anhand der Muskelaktivität und nicht zur Unterstützung eines Zusatzsensors, das Sensorsignal wird also derart ausgewertet, dass direkt aus der Muskelaktivität heraus ermittelt wird, dass der Anwender spricht und insbesondere auch was genau von dem Anwender gesprochen wird.

Alternativ oder zusätzlich wird durch die Spracherkennung eine solche Spracherkennung realisiert, bei welcher das Hörgerät über Sprachkommandos eingestellt wird, ohne die Mikrofonsignale auswerten zu müssen, welche möglicherweise Störsignale oder Fremdstimmen enthalten.

In einer bevorzugten Ausgestaltung wird anhand der Muskelaktivität ein Gesichtsausdruck des Anwenders erkannt, welcher als ein Steuerkommando zum Einstellen eines bestimmten Betriebsmodus verwendet wird, welcher dem Steuerkommando zugeordnet ist. Die Muskelaktivität dient also allgemein als Steuerkommando für das Hörgerät. Der Anwender formt einen Gesichtsausdruck, welcher eine bestimmte Muskelaktivität nach sich zieht, welche dann erkannt wird. Dadurch lassen sich auch Emotionen und somit Intentionen im weiteren Sinne erkennen und basierend darauf ein geeigneter Betriebsmodus auswählen. In einer Variante wird dann anhand der Muskelaktivität eine Emotion des Anwenders ermittelt und aus dieser Erkenntnis wiederum die Intention abgeleitet. Dabei wird ausgenutzt, dass sich die Emotion in einem bestimmten Gesichtsausdruck äußert, welcher auch die Ohrmuskeln beeinflusst

Besonders vorteilhaft ist eine Ausgestaltung, bei welcher anhand der Muskelaktivität eine Fehlpositionierung des Hörgeräts erkannt wird, falls das Sensorsignal einen Merkmalsvektor aufweist, welcher dem vorbekannten Merkmalsvektor lediglich ähnlich ist. Dadurch wird zuverlässig erkannt, ob das Hörgerät korrekt getragen wird oder ob eine Abweichung von einer vorgegebenen Trageposition vorliegt. Dabei wird unter "ähnlich" insbesondere verstanden, dass der Merkmalsvektor, welcher in dem Sensorsignal gefunden wurde, zwar eine gewisse Übereinstimmung mit dem vorbekannten Merkmalsvektor aufweist, dass die Abweichung zu diesem aber größer ist, als eine Toleranz, innerhalb welcher der Merkmalsvektor in dem Sensorsignal als übereinstimmend mit dem vorbekannten Merkmalsvektor angesehen würde. Dies ist beispielsweise der Fall, falls die beiden Merkmalsvektoren lediglich in einigen Merkmalen übereinstimmen oder falls einige oder alle der Merkmale leicht abweichende Werte aufweisen.

In einer geeigneten Ausgestaltung werden die beiden Merkmalsvektoren bezüglich ihrer Amplitude miteinander verglichen, um eine Fehlpositionierung festzustellen. Hierzu wird beispielsweise die Energie in jedem der Frequenzbänder als Merkmal verwendet. Dem liegt die Überlegung zugrunde, dass bei nicht korrekter Positionierung des Elektrodenarrays üblicherweise weiter entfernt vom Ohrmuskel liegt und dadurch das Sensorsignal insgesamt schwächer ist, sodass auch die Merkmale entsprechend schwächer ausgeprägt sind. Andersherum kann auch das Elektrodenarray derart gegenüber der üblichen Trageposition liegen, sodass dann ein insgesamt stärkeres Sensorsignal gemessen wird.

Alternativ oder zusätzlich wird die Regularität der beiden Merkmalsvektoren bestimmt und verglichen. Die Regularität gibt insbesondere an, in welcher Beziehung einzelne Frequenzkomponenten zueinander stehen, d.h. welches Verhältnis zwei Frequenzkomponenten zueinander aufweisen. Auch hierzu wird beispielsweise die Energie der einzelnen Frequenzbänder verwendet, um zunächst die Regularität eines Merkmalsvektors zu bestimmen, indem die Energien zweier Frequenzbänder ins Verhältnis gesetzt werden, und dann die Regularitäten des vorbekannten Merkmalsvektors und des Merkmalsvektors im Sensorsignal miteinander zu vergleichen. Insofern wird eine tatsächliche Regularität des Merkmalsvektors im Sensorsignal mit einer Sollregularität des vorbekannten Merkmalsvektors verglichen, um eine Fehlpositionierung zu erkennen.

Im Rahmen der Erkennung einer Fehlpositionierung ist der vorbekannte Merkmalsvektor insbesondere ein erwarteter Merkmalsvektor, d.h. bei der Untersuchung des Sensorsignals wird der vorbekannte Merkmalsvektor erwartet, jedoch ein hiervon abweichender, aber ähnlicher Merkmalsvektor gefunden. In einer zweckmäßigen Weiterbildung, wird der vorbekannte Merkmalsvektor aus mehreren vorbekannten Merkmalsvektoren als erwarteter Merkmalsvektor ausgewählt, indem zusätzlich ermittelt wird, welcher Betriebsmodus eingestellt ist oder welche Situation aktuell vorliegt, und indem derjenige vorbekannte Merkmalsvektor ausgewählt wird, welcher dem Betriebsmodus oder der Situation zugeordnet ist. Bei der Erkennung einer Fehlpositionierung wird also die Kenntnis der aktuellen Situation oder des aktuellen Betriebsmodus ausgenutzt, um festzulegen, welcher Merkmalsvektor überhaupt in dem Sensorsignal erwartet wird. Der Betriebsmodus oder die aktuelle Situation werden zweckmäßigerweise mittels eines Zusatzsensors ermittelt, z.B. eines Mikrofons, dessen Mikrofonsignal einer Audioanalyse unterzogen wird.

Die Fehlpositionierung wird in einer zweckmäßigen Weiterbildung erkannt und korrigiert, indem ein Maß für die Fehlpositionierung als ein Istwert einer Regelschleife zugeführt wird. Als Maß wird beispielsweise ein aktueller Merkmalsvektor in dem Sensorsignal verwendet oder ein bestimmter Wert für eine Anzahl von Merkmalen in dem Sensorsignal. Der vorbekannte Merkmalsvektor, welcher der optimalen Positionierung entspricht ist dann insbesondere ein Sollwert für die Regelschleife. Zweckmäßigerweise wird ein Warnhinweis ausgegeben, solange eine Fehlpositionierung vorliegt, wodurch der Anwender veranlasst werden soll die Positionierung anzupassen.

Insgesamt lassen sich also vorteilhaft beliebige Einstellungen anhand der Erkennung der Intention des Anwenders durch Messung der Muskelaktivität realisieren. Genannt wurde bereits die Einstellung einer Richtungscharakteristik. Im Rahmen der bereits beschriebenen Ausgestaltungen oder alternativ oder zusätzlich wird als Betriebsmodus ein bestimmtes Verstärkungsschema eingestellt, ein bestimmtes Kompressionsschema, d.h. Frequenzkompressionsschema, eine Rauschunterdrückung oder ein anderer Algorithmus oder eine Kombination hiervon. Solche Einstellungen sind besonders in Kombination mit der weiter oben beschriebenen Eigenstimmenerkennung von Vorteil, da in einer Eigenstimmensituation üblicherweise eine Änderung des Betriebsmodus sinnvoll ist. Beim Wechsel von oder zu einer Eigenstimmensituation werden zweckmäßigerweise entsprechende Einstellungen vorgenommen, welche z.B. wie oben beschrieben den Eigenstimmenanteil unterdrücken. Auch beim Erkennen eines Gesichtsausdrucks ist ein Einstellen oder eine Anpassung des Betriebsmodus sinnvoll, um auf die höchstwahrscheinlich veränderte Situation zu reagieren.

Die Einstellung des Hörgeräts anhand der Muskelaktivität erfolgt automatisch. Zweckmäßigerweise ist die gewählte Einstellung jedoch verhinderbar, insbesondere indem der Anwender durch eine manuelle Eingabe das Ergebnis der automatischen Erkennung überschreibt.

Vorteilhafterweise erfolgt zusätzlich zu der Dekodierung der Intention des Anwenders anhand vorbekannter Merkmalsvektoren auch eine Aufnahme, d.h. Aufzeichnung neuer Merkmalsvektoren, um neue und bisher unbekannte Intentionen zukünftig zu erkennen. Das Verfahren enthält dann also auch ein Lernverfahren, bei welchem das Hörgerät neue Merkmalsvektoren erlernt und dann als nunmehr vorbekannte Merkmalsvektoren abspeichert. Dem liegt die Erkenntnis zugrunde, dass die Muskelaktivität der Ohrmuskulatur von Anwender zu Anwender wenigstens teilweise individuell ist, dass also dieselbe Intention bei verschiedenen Anwendern durch unterschiedliche Merkmale gekennzeichnet sein kann. Umgekehrt kann grundsätzlich auch derselbe Merkmalsvektor bei unterschiedlichen Anwendern durch unterschiedliche Intentionen bedingt sein. Zweckmäßigerweise erfolgt also eine individuelle Anpassung durch Aufnahme neuer Merkmalsvektoren. In einer geeigneten Ausgestaltung wird dann eine Anzahl an neuen Merkmalsvektoren aufgenommen, d.h. aufgezeichnet, und als eine entsprechende Anzahl an vorbekannten Merkmalsvektoren gespeichert, um anschließend das Sensorsignal auf diese nunmehr vorbekannten und somit gelernten Merkmalsvektoren hin zu untersuchen.

In einer besonders bevorzugten Ausgestaltung wird zum Lernen neuer Merkmalsvektoren das Sensorsignal zusätzlich mittels eines Merkmalsextraktors einer Merkmalsextraktion unterzogen, bei welcher ein neuer und insbesondere individueller, d.h. anwenderspezifischer Merkmalsvektor erzeugt wird, welcher als ein zusätzlicher vorbekannter Merkmalsvektor gespeichert wird. Aus dem Sensorsignal wird demnach ein neuer Merkmalsvektor extrahiert und dann gespeichert, um zukünftig auf diesen als vorbekannten Merkmalsvektor zurückzugreifen. Der neue Merkmalsvektor ist dann ein gelernter Merkmalsvektor. Insgesamt definiert und lernt das Hörgerät also eine neue Klasse, welche fortan zur Klassifikation zur Verfügung steht.

Zur Aufnahme eines neuen Merkmalsvektors wird zweckmäßigerweise ein Trainingsverfahren durchgeführt. Im Rahmen des Trainingsverfahrens werden in einer Trainingssituation bestimmte Intentionen des Anwenders gewollt hervorgerufen, z.B. durch bestimmte Reize, woraus sich eine Muskelaktivität ergibt, deren Merkmale als neuer Merkmalsvektor gespeichert werden. Die zugehörige Intention ist aufgrund der Trainingssituation gezielt hervorgerufen und dadurch hinreichend bekannt, sodass eine zuverlässige Zuordnung gewährleistet ist. Das Trainingsverfahren wird in einer Variante beim Audiologen durchgeführt. Alternativ oder zusätzlich wird das Trainingsverfahren vom Anwender selbst durchgeführt, z.B. unterstützt durch eine Trainingssoftware oder einen Trainingsmodus des Hörgeräts. Das Trainingsverfahren wird geeigneterweise in verschiedenen Situationen und Umgebungen durchgeführt, um verschiedene Intentionen zusätzlich in unterschiedlichen Situationen und Umgebungen, d.h. situationsspezifisch, sicher zu erkennen.

Die vorbekannten Merkmalsvektoren, d.h. Klassen, können grundsätzlich auf verschiedene Weise vorgegeben werden. Einerseits können Merkmalsvektoren bereits bei der Herstellung des Hörgeräts als sozusagen werksseitige Merkmalsvektoren vorgegeben werden. Solche werksseitigen Merkmalsvektoren berücksichtigen jedoch prinzipbedingt nicht das individuelle Antwortverhalten der Ohrmuskeln des jeweiligen Anwenders und sind daher insbesondere beschränkt auf solche Merkmalsvektoren, welche sich unabhängig vom Anwender generalisieren lassen.

Andererseits können weitere Merkmalsvektoren wie beschrieben im Rahmen eines Trainingsverfahrens, z.B. einer individuellen Fitting Session für den Anwender hinzugefügt werden. Hierbei werden dem Anwender gezielt bestimmte Reize dargeboten und dann dessen individuelles Antwortverhalten gemessen, um dann aus dem resultierenden Sensorsignal einen neuen und individuellen Merkmalsvektor für den dargebotenen Reiz und die zugehörige Intention abzuleiten und zu speichern. In gleicher Weise werden in einer Variante bereits bekannte Merkmalsvektoren angepasst, d.h. es erfolgt eine Feineinstellung vorbekannter Merkmalsvektoren.

In einer geeigneten Ausgestaltung wird der vorbekannte Merkmalsvektor einer externen Datenbank, welche z.B. auf einem Server hinterlegt ist, entnommen. Der vorbekannte Merkmalsvektor ist also anfänglich nicht im Hörgerät gespeichert, sondern wird von diesem bei der externen Datenbank angefragt oder von dieser automatisch übermittelt. Die externe Datenbank enthält insbesondere eine Vielzahl an vorbekannten Merkmalsvektoren und dient als eine zentrale Speicherstelle. Neue Merkmalsvektoren werden zweckmäßigerweise in der externen Datenbank gespeichert und sind dann vorteilhaft auch für andere Anwender verfügbar. Dadurch kann die Lernzeit, d.h. der Aufwand bei der Extraktion neuer Merkmalsvektoren drastisch reduziert werden. Obwohl die Muskelaktivität hochgradig individuell ist, profitiert das Verfahren dennoch von den Merkmalsvektoren, welche im Zusammenhang mit einem anderen Anwender ermittelt wurden, indem beim Erkennen eines neuen Merkmalsvektors die Merkmalsvektoren eines anderen Anwenders als Startwerte zugrunde gelegt werden und diese Startwerte dann anwenderspezifisch optimiert werden. Die Speicherung der Merkmalsvektoren in einer externen Datenbank ermöglicht zudem auch eine Metaanalyse, mittels welcher vorteilhafterweise generalisierbare Merkmalsvektoren ermittelt werden, d.h. Merkmalsvektoren, welche gar nicht oder nur in geringem Maße anwenderspezifisch sind. Insofern werden dann die neuen Merkmalsvektoren einer Mehrzahl an Anwendern in der externen Datenbank zusammengeführt und generalisiert, um generalisierte Merkmalsvektoren zu ermitteln, welche dann wiederum als vorbekannte Merkmalsvektoren einem einzelnen Anwender zur Verfügung stehen und beim Betrieb von dessen Hörgerät zur Klassifikation des Sensorsignals verwendet werden.

Besonders vorteilhaft ist eine Ausgestaltung, bei welcher mittels eines Maschinenlernverfahrens selbsttätig individuelle Merkmalsvektoren gelernt werden. Dadurch werden gelernte Merkmalsvektoren erzeugt und gespeichert und bei nachfolgenden Analysen als nunmehr vorbekannte Merkmalsvektoren mit berücksichtigt. Die Analyse der Muskelaktivität eignet sich ganz besonders für ein Maschinenlernverfahren, da die Muskelaktivität hochgradig individuell ist und damit nur beschränkt generalisierbar. Demgegenüber ist beispielsweise die Klassifikation von Geräuschumgebungen anhand von Mikrofonsignalen sehr einfach generalisierbar, da eine jeweilige Geräuschumgebung durch bestimmte Geräusche und somit durch einen a priori bekannten Merkmalsvektor charakterisiert ist. Das durch Vermittlung des Gehirns des Anwenders individuell erzeugte Antwortverhalten des Ohrmuskels erzeugt jedoch in den wenigsten Fällen für eine bestimmte Situation einen Merkmalsvektor, welcher unabhängig vom Anwender gleich ist. Bei dem Maschinenlernverfahren wird dann ein anwenderspezifisches Set an Merkmalsvektoren erzeugt.

Das Maschinenlernverfahren wird von einer Lernmaschine ausgeführt. Diese ist vorzugsweise ein Teil einer Steuereinheit des Hörgeräts und besonders bevorzugt in ein Gehäuse des Hörgeräts integriert. Alternativ oder zusätzlich ist die Lernmaschine extern angeordnet, z.B. auf einem Smartphone des Anwenders oder auf einem Server.

Ein besonders geeignetes Maschinenlernverfahren zur Merkmalsextraktion ist in Strauss and Steidl "Hybrid wavelet-support vector classification of waveforms", Journal of Computational and Applied Mathematics 148 (2002), p.375-400 beschrieben. Das dort beschriebene Verfahren wird vorzugsweise ebenfalls zur Klassifikation, d.h. zur Erkennung von vorbekannten Merkmalsvektoren, insbesondere zuvor durch Merkmalsextraktion gelernten Merkmalsvektoren, im Sensorsignal verwendet. Demnach ist der Klassifikator vorzugsweise durch eine support vector machine (kurz: SVM) implementiert, welche im Rahmen des Maschinenlernverfahrens die unterschiedlichen Merkmalsvektoren, d.h. Klassen, welche unterschiedlichen Betriebsmodi zugeordnet sind, maximal gegeneinander diskriminiert und dadurch eine besonders sichere Klassifizierung, d.h. Erkennung der vorbekannten Merkmalsvektoren gewährleistet. Dieser Vorteil kommt zugleich auch der Merkmalsextraktion zugute, da entsprechend neu gelernte Merkmalsvektoren automatisch optimal unterscheidbar sind.

In einer bevorzugten Ausgestaltung wird der neue Merkmalsvektor in einem Maschinenlernverfahren mit Unterweisung erzeugt. Die Lernmaschine wird also in einem Lernverfahren trainiert. Einer bestimmten Situation mit einem noch unbekanntem Merkmalsvektor ist ein bestimmter Betriebsmodus zugeordnet. Der an sich bekannte Betriebsmodus für die an sich bekannte Situation soll nun durch Erkennung der Muskelaktivität einstellbar gemacht werden, wobei aber die insbesondere individuelle Muskelaktivität noch unbekannt ist. Im Rahmen des Maschinenlernverfahrens wird nunmehr insbesondere in einem Trainingsverfahren die bestimmte Situation als die aktuelle Situation hergestellt und dadurch eine entsprechende Intention des Anwenders hervorgerufen, welche dann durch einen Merkmalsvektor charakterisiert ist, welcher bestimmt wird und mit dem Betriebsmodus verknüpft wird. Hierzu sucht der Merkmalsextrator eigenständig eine Anzahl an unbekannten Merkmalen in dem Sensorsignal. Die unbekannten Merkmale werden dann zu dem neuen Merkmalsvektor zusammengefasst und dieser dem bestimmten Betriebsmodus zugeordnet. Der neue Merkmalsvektor ist dann ein gelernter Merkmalsvektor und steht fortan als vorbekannter Merkmalsvektor zur Verfügung.

Wesentlich an dem Maschinenlernverfahren ist, dass die Merkmale nicht anfänglich vorgegeben sind, sondern vom Merkmalsextraktor eigenständig ermittelt werden. Der Merkmalsextraktor untersucht hierzu das Sensorsignal nach Mustern oder Regelmäßigkeiten und insbesondere nach Merkmalen, welche in Kombination keinem schon vorbekannten Merkmalsvektor entsprechen. In einer Variante wird dagegen explizit nach Merkmalen gesucht, welche einem vorbekannten Merkmalsvektor ähneln, um dessen Repräsentation zu verfeinern oder anzupassen. Die Situation, welche hierzu eingestellt wird, ist zweckmäßigerweise ebenjene Situation welche über die zugehörige Intention dem zu verbessernden Merkmalsvektor zugeordnet ist.

Besonders vorteilhaft ist eine Ausgestaltung, bei welcher ein Maschinenlernverfahren, insbesondere wie oben beschrieben, im Normalbetrieb des Hörgeräts durchgeführt wird, d.h. beim Tragen im Alltag und außerhalb einer speziellen Trainingssituation. Der Merkmalsextraktor untersucht dann fortlaufend, ob im Sensorsignal neue Merkmale oder neue Merkmalsvektoren enthalten sind, welche noch nicht vorbekannt sind. Zugleich werden die aktuelle Situation oder der Betriebsmodus, welcher aktuell eingestellt ist, oder beides erfasst und dem Merkmalsvektor zugeordnet. Dem liegt die Überlegung zugrunde, dass die jeweilige Situation reproduzierbar eine bestimmte Intention des Anwenders erzeugt und einen bestimmten Betriebsmodus erfordert. Durch Aufnahme des Merkmalsvektors, welcher charakteristisch für diese Situation oder den Betriebsmodus oder beides ist, wird dann zukünftig zuverlässig die Situation wieder erkannt und der geeignete Betriebsmodus eingestellt.

Geeigneterweise wird im Rahmen einer hybriden Analyse mittels eines Zusatzsensors erkannt, ob die aktuelle Situation eine vorbekannte Situation ist, welcher bereits ein vorbekannter Betriebsmodus zugeordnet ist, und der neue Merkmalsvektor wird dem vorgekannten Betriebsmodus zugeordnet, falls die aktuelle Situation der vorbekannten Situation entspricht. Damit ist insbesondere ein Lernverfahren realisiert, bei welchem das Hörgerät selbstständig neue Zuordnungen zwischen der Muskelaktivität und der aktuellen Situation erzeugt. Das Problem der Zuordnung des Merkmalsvektors zu einer bestimmten Situation und zu einem bestimmten Betriebsmodus wird durch einen Zusatzsensor gelöst, sodass das Verfahren auch außerhalb einer Trainingssituation durchführbar ist, d.h. die Situation muss nicht von außerhalb identifiziert werden. Vielmehr wird der Zusatzsensor verwendet, um die aktuelle Situation zu klassifizieren und den optimalen Betriebsmodus zu identifizieren. Der benötigte Betriebsmodus ist dann bekannt und kann dem neuen Merkmalsvektor zugeordnet werden. Der Zusatzsensor ist beispielsweise ein Mikrofon, welcher ein Mikrofonsignal erzeugt, welches einer Audioanalyse unterzogen wird, um die aktuelle Situation zu klassifizieren und einen geeigneten Betriebsmodus auszuwählen. Nachdem dann für diese Situation die Muskelaktivität und ein entsprechender Merkmalsvektor gelernt wurden, erfolgt eine zukünftige Erkennung entsprechend schneller und einfacher, ohne auf eine aufwendige Audioanalyse zurückgreifen zu müssen. Alternativ oder zusätzlich wird eine EEG-Messung durchgeführt, der Zusatzsensor ist dann eine Anzahl an EEG-Elektroden. Geeignet ist auch ein Beschleunigungssensor als Zusatzsensor.

In einer vorteilhaften Ausgestaltung ist der vorbekannte Merkmalsvektor in ein neuronales Netz integriert, mittels welchem untersucht wird, ob das Sensorsignal einen vorbekannten Merkmalsvektor aufweist, indem ein Merkmalsvektor des Sensorsignals bestimmt wird und als ein Eingangssignal für das neuronale Netz verwendet wird. Ein wesentlicher Vorteil eines neuronalen Netzes besteht insbesondere darin, dass dieses nicht zwingend in einem Lernverfahren mit Unterweisung trainiert werden muss, sondern auch ohne Unterweisung lernen und arbeiten kann. Ein vorbekannter Merkmalsvektor ist dann nicht in einem Speicher hinterlegt, sondern in die Topologie des neuronalen Netzes integriert.

Besonders im Zusammenhang mit der zuvor beschriebenen Ausgestaltung mit einem Zusatzsensor ist also auch ein Maschinenlernverfahren ohne Unterweisung realisierbar ist, d.h. die Lernmaschine muss nicht trainiert werden, sondern lernt vollständig selbsttätig unter Zuhilfenahme des Zusatzsensors. Auf einen separaten Speicher für die vorbekannten Merkmalsvektoren wird insbesondere verzichtet. Vielmehr werden diejenigen Merkmalsvektoren, welche in das neuronale Netz integriert sind, nunmehr konkreten Betriebsmodi zugeordnet. Mit anderen Worten: zunächst wird mittels eines Zusatzsensors erkannt, ob die aktuelle Situation eine vorbekannte Situation ist, welcher bereits ein vorbekannter Betriebsmodus zugeordnet ist. Falls dann das neuronale Netz den Merkmalsvektor des Sensorsignals erkennt, d.h. falls in das neuronale Netz ein Merkmalsvektor integriert ist, welcher mit dem Merkmalsvektor des Sensorsignals übereinstimmt, wird dieser Merkmalsvektor dem vorbekannten Betriebsmodus zugeordnet. Unter Zuhilfenahme des Zusatzsensors wird also in einem Maschinenlernverfahren ohne Unterweisung eine Zuordnung eines integrierten Merkmalsvektors zu einem Betriebsmodus erzeugt, indem mittels des Zusatzsensor die aktuelle Situation bestimmt wird und idem dann der integrierte Merkmalsvektor dieser aktuellen Situation zugeordnet wird und somit auch dem für diese Situation geeigneten Betriebsmodus, welcher dann zukünftig eingestellt wird, wenn der nunmehr gelernte, d.h. zugeordnete Merkmalsvektor erneut im Sensorsignal gefunden wird. Der integrierte Merkmalsvektor wird also durch das Lernverfahren zu einem vorbekannten Merkmalsvektor.

Analog zum Maschinenlernverfahren mit Unterweisung wird auch das maschinenlernverfahren ohne Unterweisung zweckmäßigerweise im Normalbetrieb des Hörgeräts durchgeführt. Bei der Ausgestaltung mit einem neuronalen Netz lernt dieses dann selbstständig, die integrierten Merkmalsvektoren jeweils einem bestimmten Betriebsmodus zuzuordnen. Dies erfolgt beispielsweise über einen Zusatzsensor wie oben beschrieben oder durch eine Rückmeldung des Anwenders.

In einer besonders geeigneten Ausgestaltung sind der Merkmalsextraktor und der Klassifikator gemeinsam in eine Signalverarbeitung integriert und die Merkmalsextraktion und die Klassifikation werden als ein einzelnes Optimierungsproblem behandelt. Der Klassifikator und der Merkmalsextraktor verwenden dann gemeinsam eine einzelne Merkmalserkennung, welche in dem Sensorsignal eine Anzahl an Merkmalen und damit einen Merkmalsvektor identifiziert. Dadurch ist die gesamte Auswertung des Sensorsignals einerseits besonders kompakt, andererseits werden vorteilhafte Synergien genutzt, welche sich bei der kombinierten Klassifikation und Merkmalsextraktion ergeben, denn die Merkmale brauchen lediglich einmal aus dem Sensorsignal extrahiert und mit den vorbekannten Merkmalsvektoren verglichen zu werden, um dann einerseits neue Merkmalsvektoren zu erzeugen und andererseits vorbekannte Merkmalsvektoren zu erkennen. Der signalverarbeitungstechnische Aufwand ist dadurch auf ein Minimum reduziert. Der Merkmalsextraktor wird zudem vorteilhaft derart betrieben, dass dieser solche Merkmale und somit solche Merkmalsvektoren extrahiert, welche für den Klassifikator besonders einfach zu erkennen sind. Da der Klassifikator und der Merkmalsextraktor dieselbe Merkmalserkennung nutzen, sind beide optimal aufeinander abgestimmt.

Das Sensorsignal wird vorzugsweise mittels einer Filterbank auf mehrere Frequenzbänder aufteilt, wobei jedes Frequenzband eine Anzahl an Merkmalen bildet. Die oben bereits beschriebene Untersuchung von Merkmalen anhand von Entropie und Energie einzelner Frequenzbänder erfolgt dann mittels der Filterbank derart, dass für jedes der Frequenzbänder eine Entropiemessung oder eine Energiemessung oder beides durchgeführt wird. Eine jeweilige Entropie- oder Energiemessung stellt dabei ein Merkmal dar und die Merkmale bilden gemeinsam einen Merkmalsvektor des Sensorsignals. Eine Filterbank ist besonders effizient und eignet sich insbesondere auch zur Behandlung der Merkmalsextraktion und der Klassifikation als ein einzelnes Optimierungsproblem. Da die Merkmale zur Bildung neuer Merkmalsvektoren gerade derjenigen Filterbank entnommen werden, welche auch zur Klassifikation verwendet wird, werden die einmal gelernten Merkmalsvektoren besonders zuverlässig wiedererkannt.

Das Maschinenlernverfahren wird vorzugsweise mit dem in Strauss and Steidl (2002, a.a.O.) oder auch in Yger and Rakotomamonjy "Wavelet kernel learning" Pattern Recognition 44, 2011, S.2614-2629 beschriebenen hybriden Wavelet-Kernel Lernalgorithmus durchgeführt. Der Lernalgorithmus basiert auf einer dynamischen Anpassung der zugrundeliegenden Wavelets, um optimale Merkmale zu extrahieren. Dies ist vorliegend besonders vorteilhaft, da die Merkmale an sich noch nicht zwingend bekannt und definiert sind und zudem typischerweise auch anwenderspezifisch sind. Insofern wird bei dem Maschinenlernverfahren also vorteilhafterweise auch die Definition der Merkmale, d.h. konkret die Filterbank angepasst, z.B. hinsichtlich der Grenzfrequenzen der einzelnen Frequenzbänder. Die Filterbank ist dabei also eine parametrisierte Filterbank. Die auf diese Weise definierten Merkmale und die hierbei konfigurierte Filterbank sind dann automatisch auch optimal für die Klassifikation geeignet also auch zur Untersuchung des Sensorsignals mittels der oben beschriebenen Signalverarbeitung.

Vorzugsweise ist die Filterbank eine paraunitäre Filterbank. Eine Definition einer paraunitären Filterbank mit zwei Frequenzbändern ist in Strauss and Steidl (2002, a.a.O.) auf S.382 gegeben, insbesondere in den Gleichungen (18) und (19). Eine besonders geeignete Filterbank mit Kreuzgliedstruktur (Englisch: lattice structure) ist dort in Gleichung (22) beschrieben. Der dort genannte Winkel dient als Parameter der Filterbank, welche dann eine parametrisierte und somit vorteilhaft anpassbare Filterbank ist.

In einer geeigneten Ausgestaltung weist die Filterbank zehn Frequenzbänder auf. Die Frequenzbänder der Filterbank sind vorzugsweise jeweils oktavbreit. Darunter wird insbesondere verstanden, dass jedes Frequenzband eine obere Bandgrenzfrequenz und eine untere Bandgrenzfrequenz aufweist, wobei die obere Bandgrenzfrequenz doppelt so groß ist, wie die untere Bandgrenzfrequenz. Dabei müssen die Frequenzbänder nicht exakt oktavbreit sein, vielmehr ist eine Abweichung um bis zu 10% ebenfalls noch geeignet. Besonders zweckmäßig ist eine Ausgestaltung, bei welcher die Bandgrenzfrequenzen wie oben bereits angedeutet mittels einer Parametrisierung angepasst werden, indem die Bandgrenzfrequenzen in Bezug auf die einzelnen Merkmale derart gewählt werden, dass die Merkmale besonders deutlich sind.

Die Filterbank weist in einer geeigneten Ausgestaltung eine untere Grenzfrequenz von 4Hz auf und eine obere Grenzfrequenz von 1kHz, sodass die Muskelaktivität in einem Frequenzbereich von 4Hz bis 1kHz untersucht wird.

In einer geeigneten Ausgestaltung wird bei einem binauralen Hörgerät eine einzelne Filterbank zur Analyse beider Sensorsignale verwendet. Die Filterbank wird dann mittels des Maschinenlernverfahrens insbesondere auch derart trainiert, dass die Filterbank die beiden Richtungen unterscheidet, d.h. dass die Filterbank eine Anzahl an Merkmalen extrahiert, welche jeweils der linken oder der rechten Seite zugeordnet werden. Alternativ werden jedoch zwei separate Filterbänke verwendet, nämlich eine für jedes Einzelgerät.

Zur Erzeugung des Sensorsignals ist das Elektrodenarray wenigstens bipolar ausgebildet, d.h. weist wenigstens zwei Elektroden auf, zwischen welchen dann eine Potentialdifferenz gemessen wird. Geeignet ist grundsätzlich auch ein Elektrodenarray mit mehr als zwei Elektroden. Für EMG-Messungen im klinischen Umfeld werden beispielsweise 128 Elektroden verwendet, welche über den gesamten Kopf verteilt angeordnet sind. Eine der Elektroden wird dann zweckmäßigerweise als eine Referenzelektrode verwendet, welche ein Referenzpotential bereitstellt, gegen welches mit den anderen Elektroden jeweils in einer bipolaren Anordnung gemessen wird. Mit anderen Worten: es wird die Potentialdifferenz zwischen jeweils einer der Elektroden und der Referenzelektrode gemessen.

Vorliegend ist das Elektrodenarray möglichst kompakt ausgebildet. Das Elektrodenarray ist dazu in einer geeigneten Ausgestaltung lediglich im Bereich der Ohrmuskulatur angeordnet, d.h. in diesem Zusammenhang höchstens 5cm vom Ohr, genauer von der Ohrmuschel entfernt, besonders bevorzugt höchstens 2cm. Dadurch ist gewährleistet, dass das Elektrodenarray lediglich über einen kleinen Teil des Kopfes des Anwenders verteilt ist und dadurch besonders alltagstauglich ist.

In einer bevorzugten Ausgestaltung weist das Elektrodenarray lediglich höchstens fünf, besonders bevorzugt genau zwei Elektroden auf. Auch eine solche Beschränkung der Elektrodenzahl trägt zur Kompaktheit des Elektrodenarray und zur Alltagstauglichkeit des Hörgeräts und des Verfahrens bei. Der Reduzierung der Elektrodenzahl vor allem im Vergleich zum klinischen Umfeld liegt insbesondere die Beobachtung zugrunde, dass zur Messung der Muskelaktivität der Ohrmuskulatur eine eingeschränkte Anzahl an Elektroden völlig ausreichend ist. Besonders in Verbindung mit einem Maschinenlernverfahren ist lediglich eine kleine Anzahl an Elektroden notwendig, um hinreichend genau die Muskelaktivität messen zu können und dabei zwischen verschiedenen Klassen ausreichend differenzieren zu können.

Bei einem binauralen Hörgerät mit zwei Einzelgeräten sind entsprechend vorzugsweise dann zwei Elektrodenarrays angeordnet, nämlich eines für jedes der Einzelgeräte. Die Verwendung lediglich eines Elektrodenarrays pro Einzelgerät mit lediglich wenigen Elektroden wie oben beschrieben wird erst durch das Konzept der EMG-Messung ermöglicht, da hier wie oben beschrieben ein ausreichend gefiltertes und verstärktes Signal vorliegt. Das Elektrodenarray ist zudem jeweils am Ohrmuskel platziert und damit ohnehin in der Nähe des Hörgeräts. Zweckmäßigerweise ist das Elektrodenarray in das Hörgerät integriert, insbesondere als Teil eines Gehäuses des Hörgeräts. Dadurch ist der Tragekomfort für den Anwender deutlich erhöht. Auch die optische Erscheinung ist höchstens minimal beeinträchtigt. Beides trägt zu einer deutlich höheren Akzeptanz bei einer Anwendung im Alltag bei. Dies ermöglicht vor allem auch eine EMG-Messung nicht lediglich im Rahmen einer Fitting Session beim Audiologen unter Verwendung von aufwändiger Apparatur, sondern vielmehr unbemerkt und unsichtbar im Alltag, d.h. im Normalbetrieb des Hörgeräts.

Zweckmäßigerweise weist das Elektrodenarray zusätzlich zu den Elektroden noch eine Elektrode auf, welche eine Referenzelektrode ist, um insbesondere die Funktionalität der übrigen Elektroden zu überwachen und eine Referenz für das Signal der Elektroden bereitzustellen. Mit anderen Worten: das Elektrodenarray weist mehrere Elektroden auf, von welchen eine als Referenzelektrode ausgebildet ist. Mittels der Referenzelektrode wird dann ein Referenzsignal abseits jeglicher Ohrmuskel des Anwenders gemessen. Mittels des Referenzsignals wird dann das Sensorsignal aufbereitet, z.B. von einem Hintergrundrauschen befreit oder normalisiert, bevor das Sensorsignal dem Klassifikator und insbesondere auch dem Merkmalsextraktor zur Merkmalsanalyse zugeführt wird. Besonders geeignet ist eine Anbringung der Referenzelektrode am Mastoid, d.h. am Warzenteil des Schläfenbeins. Dies gewährleistet eine hinreichende Referenz. Weiter kann die Referenzelektrode dort weitgehend unbemerkt getragen werden. Allgemein ist die Referenzelektrode geeigneterweise ebenfalls in der Nähe des Ohrs anbringbar und im Betrieb auch angebracht, jedoch gerade nicht an einem der Ohrmuskel.

Besonders vorteilhaft ist eine Kombination der obigen Ausgestaltungen derart, dass das Elektrodenarray genau eine Elektrode und eine Referenzelektrode aufweist, also insgesamt lediglich zwei Elektroden, welche beide in das Gehäuse des Hörgeräts integriert sind und somit jeweils nahe des Ohrs des Anwenders angeordnet sind.

Die Elektroden des Elektrodenarrays und gegebenenfalls auch die Referenzelektrode sind vorzugsweise jeweils als Außenelektrode ausgebildet, d.h. als Elektrode, welche außerhalb des Kopfs des Anwenders angeordnet sind. Alternativ ist auch eine Ausgestaltung einer oder mehrerer der Elektroden als Implantat geeignet.

Eine jeweilige Elektrode ist wie oben beschrieben in einer geeigneten Variante in ein Gehäuse des Hörgeräts integriert. Geeignet ist jedoch auch eine Ausgestaltung als separate Elektrode, welche dann über eine Signalleitung oder drahtlos angebunden ist, um die Signale zur Signalverarbeitung zu übertragen.

Zweckmäßigerweise ist das Elektrodenarray zur Messung am Ohrmuskel auch an ebenjenem Ohrmuskel angebracht, liegt also z.B. außen auf der Kopfhaut auf und misst dann direkt am Ohrmuskel, welcher unter der Kopfhaut liegt. In einer Alternative oder in Kombination zu Vorgenanntem ist das Elektrodenarray dagegen im Gehörgang angeordnet, also noch außerhalb des Kopfes und nicht als Implantat ausgebildet, aber in den Gehörgang eingesetzt. Dem liegt der Gedanke zugrunde, dass auch hier noch das Antwortverhalten des Ohrmuskels messbar ist. Eine solche Ausgestaltung eignet sich besonders für Im-Ohr-Hörgeräte, also solche Hörgeräte, welche ganz oder teilweise im Gehörgang getragen werden.

Das Hörgerät weist eine Steuereinheit auf, welche zur Durchführung eines Verfahrens wie oben beschrieben ausgebildet ist. Vorzugsweise sind der Klassifikator und der Merkmalsextraktor ein Teil der Steuereinheit. Zweckmäßigerweise ist die Lernmaschine ein Teil der Steuereinheit. Der Klassifikator, der Merkmalsextraktor oder die Lernmaschine oder eine Kombination hiervon sind in einer Variante dagegen auf einem externen Gerät, z.B. einem Smartphone untergebracht, welches mit dem Hörgerät zur Signalübertragung verbunden ist. Das externe Gerät dient dann der Entlastung des Hörgeräts, da dieses unter Umständen eine lediglich begrenzte Rechenleistung aufweist.

Vorzugsweise ist das Elektrodenarray ein Teil des Hörgeräts, alternativ ist das Elektrodenarray als externer Sensor konzipiert und wird z.B. mittels eines Kabels oder einer Drahtlosverbindung an das Hörgerät gekoppelt.

Das Hörgerät ist vorzugsweise ein sogenanntes BTE-Gerät, welches hinter dem Ohr getragen wird. Dies schließt sogenannte RIC-Geräte ein, bei welchen der Hörer in den Gehörgang eingesetzt ist, das übrige Hörgerät jedoch außerhalb davon getragen wird. Andere Bauformen wie z.B. ITO (in dem Ohr) oder CIC (vollständig im Gehörgang) sind jedoch grundsätzlich auch geeignet.

Die Erfindung ist jedoch nicht auf ein Hörgerät zur Versorgung einer hörgeschädigten Person beschränkt. In einer ebenfalls geeigneten Ausgestaltung ist das Hörgerät ein Kopfhörer oder ein ähnliches Gerät zur Schallausgabe. Wesentlich ist, dass das Hörgerät einen Hörer zur Schallausgabe aufweist.

Nachfolgend wird ein Ausführungsbeispiel der Erfindung anhand einer Zeichnung näher erläutert. Darin zeigen jeweils schematisch:
- Fig. 1: ein Ohr eines Anwenders und ein Hörgerät,
- Fig. 2: das Hörgerät aus Fig. 1,
- Fig. 3: eine Signalverarbeitung des Hörgeräts aus Fig. 1,
- Fig. 4: ein Maschinenlernverfahren für das Hörgerät aus Fig. 1, und
- Fig. 5: ein Verfahren zum Einstellen des Hörgeräts aus Fig. 1.

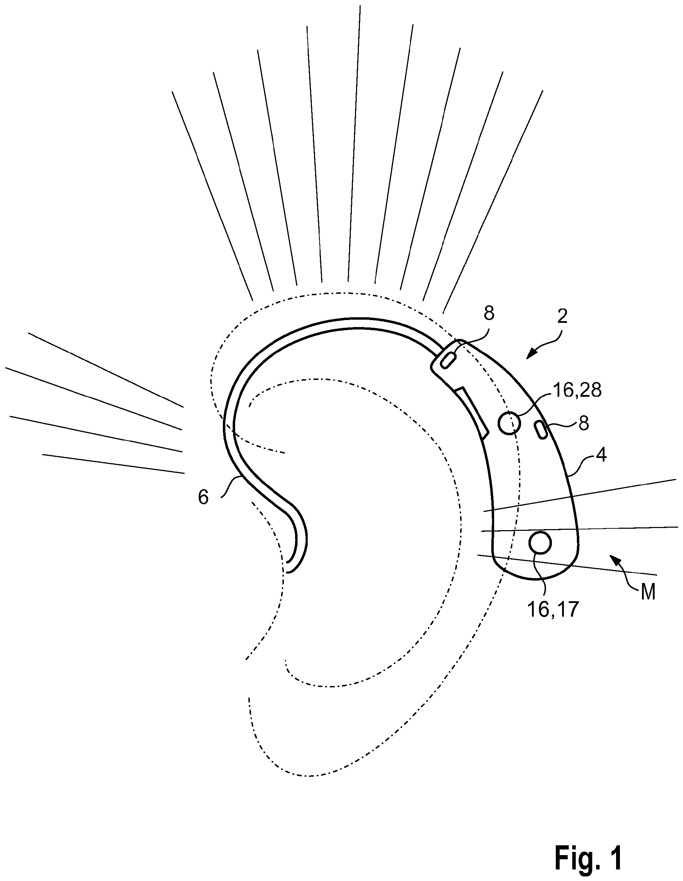

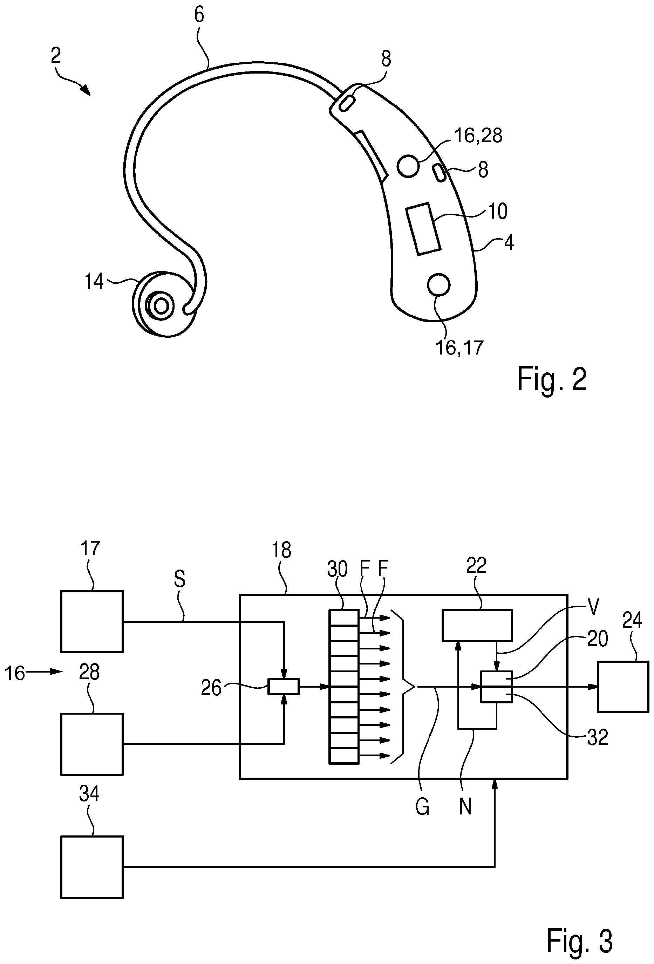

In Fig. 1 ist ein Hörgerät 2 dargestellt, welches hinter einem Ohr O eines nicht weiter dargestellten Anwenders getragen wird. Das Hörgerät 2 ist in Fig. 2 näher dargestellt. Das Hörgerät 2 ist hier ein BTE-Hörgerät, mit einem Gehäuse 4, welches hinter dem Ohr O getragen wird und von welchem ausgehend sich ein Schallschlauch 6 bis in den Gehörgang erstreckt. Das Hörgerät 2 weist weiterhin eine Anzahl an Mikrofonen 8 auf, welche Schallsignale aus der Umgebung des Anwenders aufnehmen. Diese Schallsignale werden dann mittels einer Steuereinheit 10 modifiziert, insbesondere verstärkt, und dann über einen Hörer 12 ausgegeben. Vom Hörer 12 aus gelangen die modifizierten Schallsignale dann über den Schallschlauch 6 in das Ohr O. Zum sicheren Halt des Schallschlauchs 6 ist endseitig an diesem ein Ohrstück 14 angebracht, welches in den Gehörgang eingesetzt wird.

Das Hörgerät 2 wird mittels eines Verfahrens betrieben, bei welchem eine Elektromyographie (kurz EMG) durchgeführt wird, wobei mittels eines Elektrodenarrays 16 eine Muskelaktivität eines Ohrmuskels M des Anwenders gemessen wird. Das Elektrodenarray 16 weist vorliegend eine einzelne Elektrode 17 auf sowie eine Referenzelektrode 28, insgesamt also zwei Pole. Der Ohrmuskel M ist hier der hintere Ohrmuskel, d.h. der musculus auricularis posterior. Ebenfalls eingezeichnet, jedoch nicht explizit bezeichnet, sind die beiden anderen extrinsischen Ohrmuskeln, nämlich der vordere und der obere Ohrmuskel. Das Elektrodenarray 16 ist hier ein Teil des Gehäuses 4 und an diesem derart positioniert, dass das Elektrodenarray 16 am hinteren Ohrmuskel M anliegt und so dessen Muskelaktivität messen kann. Die Muskelaktivität des Ohrmuskels M wird fortlaufend gemessen, sodass das Aktivitätsprofil des Ohrmuskels M erfasst wird. Das Aktivitätsprofil ist vergleichsweise komplex, d.h. der Ohrmuskel M führt nicht lediglich einzelne und zusammenhanglose Regungen aus. Vielmehr ist in dem Aktivitätsprofil eine Intention des Anwenders kodiert, sodass in der Muskelaktivität über einen längeren Messzeitraum von z.B. einer Sekunde hinweg komplexe Muster enthalten sind. Diese werden im Rahmen des Verfahrens dekodiert, um das Hörgerät 2 optimal einzustellen, d.h. einen für die aktuelle Situation geeigneten Betriebsmodus einzustellen.

Das Elektrodenarray 16 erzeugt ein Sensorsignal S, welches einer Signalverarbeitung 18 zugeführt wird, welche in Fig. 3 näher gezeigt ist. Die Signalverarbeitung 18 ist ein Teil der Steuereinheit 10. Die Signalverarbeitung 18 weist einen Klassifikator 20 auf, mittels welchem das Sensorsignal S einer Klassifikation unterzogen wird. Dabei wird die Muskelaktivität dekodiert und die zugrunde liegende Intention des Anwenders bestimmt, indem untersucht wird, ob das Sensorsignal S einen vorbekannten Merkmalsvektor V aufweist. Hierzu ist in einem Speicher 22 der Signalverarbeitung 18 eine Anzahl an vorbekannten Merkmalsvektoren V gespeichert, welche nunmehr in dem Sensorsignal S gesucht werden. Jedem vorbekannten Merkmalsvektor V ist ein Betriebsmodus des Hörgeräts 2 zugeordnet, welcher dann eingestellt wird, wenn das Sensorsignal S den vorbekannten Merkmalsvektor V aufweist. Vorliegend wird eine Richtungscharakteristik 24 eingestellt, d.h. das Hörgerät 2 ist zum Richtungshören ausgebildet und weist eine Richtkeule auf, mit einem Winkel und einer Breite, welche in Abhängigkeit der Muskelaktivität und der darin kodierten Intention, welche vorliegend eine Hörintention ist, ausgewählt und eingestellt werden.

Der Vorgang ist in Fig. 3 verdeutlicht. Das Sensorsignal S des Elektrodenarrays 16 wird der Signalverarbeitung 18 zugeführt und dort zunächst mittels eines Vorfilters 26 vorgefiltert. Hierzu wird ein Sensorsignal einer Referenzelektrode 28 verwendet, welche ein Teil des Elektrodenarrays 16 ist und ebenfalls ein Teil des Gehäuses 6 ist, jedoch abseits der Ohrmuskeln positioniert ist und somit ein Referenzsignal liefert. Zusätzlich werden in dem Vorfilter 26 Rauschartefakte eliminiert. Das aufbereitete Sensorsignal S wird dann einer Filterbank 30 zugeführt, mittels welcher das Sensorsignal S in einzelne Merkmal F zerlegt wird. Die Filterbank 30 weist hier zehn Frequenzbänder auf. In Fig. 3 ergibt sich für jedes Frequenzband ein Merkmal F. Ein jeweiliges Merkmal F ist beispielsweise die Energie in dem betreffenden Frequenzband, gemessen über den Messzeitraum, oder die Entropie des Frequenzbands. In einer Variante werden mehrere Merkmale F pro Frequenzband erzeugt, z.B. jeweils Entropie und Energie. Die Merkmale F bilden einen Merkmalsvektor G, welcher ein Merkmalsvektor G des Sensorsignals S ist und welcher im Klassifikator 20 mit den vorbekannten Merkmalsvektoren V verglichen wird. Auf Basis des Ergebnisses wird dann die Richtcharakteristik 24 eingestellt. Dabei wird die Erkenntnis ausgenutzt, dass die Muskelaktivität evolutionsbiologisch eine Ausrichtung der Ohren O bewirken soll, insofern ist in der Muskelaktivität der Ohrmuskeln M eine Intention zum Hören in einer bestimmten Richtung kodiert. Eine Ausrichtung der Ohren O findet tatsächlich nicht statt, die Steuersignale an die Ohrmuskeln M sind jedoch vorhanden und weisen eine entsprechende Signatur auf, welche ein entsprechendes Aktivitätsprofil ergibt und welche üblicherweise anwenderspezifisch ist. Durch Kenntnis dieser Signatur, d.h. des zugehörigen Merkmalsvektors G, wird dann die Intention eines Richtungshörens erkannt, vorliegend sogar nicht lediglich die Tatsache, dass ein Richtungshören gewünscht ist, sondern vielmehr auch die gewollte Hörrichtung erkannt.

Zusätzlich zu der Dekodierung der Intention des Anwenders anhand vorbekannter Merkmalsvektoren V erfolgt vorliegend auch eine Aufnahme, d.h. Aufzeichnung neuer Merkmalsvektoren N, um neue und bisher unbekannte Intentionen zukünftig zu erkennen. Das Verfahren ist somit auch ein Lernverfahren, bei welchem das Hörgerät 2 neue Merkmalsvektoren N erlernt und dann als vorbekannte Merkmalsvektoren V abspeichert. Hierzu wird das Sensorsignal S mittels eines Merkmalsextraktors 32 einer Merkmalsextraktion unterzogen, bei welcher ein neuer und insbesondere individueller, d.h. anwenderspezifischer Merkmalsvektor N erzeugt wird, welcher als ein zusätzlicher vorbekannter Merkmalsvektor V im Speicher 22 gespeichert wird.

Vorliegend sind der Merkmalsextraktor 32 und der Klassifikator 18 gemeinsam in die Signalverarbeitung 18 integriert und die Merkmalsextraktion und die Klassifikation werden als ein einzelnes Optimierungsproblem behandelt. Wie deutlich in Fig. 3 gezeigt ist verwenden der Klassifikator 18 und der Merkmalsextraktor 32 gemeinsam eine einzelne Merkmalserkennung, welche in dem Sensorsignal S eine Anzahl an Merkmalen F und damit ein Merkmalsvektor G identifiziert. Die Merkmale G brauchen lediglich einmal mittels der Filterbank 30 aus dem Sensorsignal S extrahiert und mit den vorbekannten Merkmalsvektoren V verglichen zu werden zu werden, um dann einerseits neue Merkmalsvektoren N zu erzeugen und andererseits vorbekannte Merkmalsvektoren V zu erkennen. Da der Klassifikator 18 und der Merkmalsextraktor 32 dieselbe Filterbank 30 zur Merkmalserkennung nutzen, sind beide optimal aufeinander abgestimmt.

Bei dem gezeigten Ausführungsbeispiel werden neue Merkmalsvektoren N mittels eines Maschinenlernverfahrens selbsttätig gelernt. Die Analyse der Muskelaktivität eignet sich ganz besonders für ein Maschinenlernverfahren, da die Muskelaktivität hochgradig individuell ist und damit nur beschränkt generalisierbar. Durch das Maschinenlernverfahren wird dann durch Erlernen neuer Merkmalsvektoren N ein anwenderspezifisches Set an vorbekannten Merkmalsvektoren V erzeugt.

Zum Anlernen neuer Merkmalsvektoren N wird das Maschinenlernverfahren überwacht durchgeführt. Hierbei wird in einem Trainingsverfahren eine bestimmte Situation als die aktuelle Situation hergestellt und dadurch eine entsprechende Intention des Anwenders hervorgerufen, welche dann durch einen Merkmalsvektor G charakterisiert wird und mit dem Betriebsmodus verknüpft wird. Hierzu sucht der Merkmalsextrator 32 eigenständig eine Anzahl an unbekannten Merkmalen F in dem Sensorsignal S. Die unbekannten Merkmale S werden dann zu dem neuen Merkmalsvektor N zusammengefasst und dieser dem bestimmten Betriebsmodus zugeordnet. Der neue Merkmalsvektor N ist dann ein gelernter Merkmalsvektor und steht fortan als vorbekannter Merkmalsvektor V im Speicher 22 zur Verfügung.

Der Speicher 22 ist vorliegend aufgrund der Verwendung einer SVM angeordnet. Bei Anwendung eines anderen Maschinenlernkonzept kann jedoch unter Umständen auf den Speicher 22 verzichtet werden. So wird in einer nicht gezeigten Variante anstelle einer SVM ein neuronales Netz verwendet, welches nicht aufgespeicherte Merkmalsvektoren V zurückgreifen muss, da diese bereits bei der Implementierung des neuronalen Netzes durch dessen Struktur gegeben sind.

Wesentlich an dem Maschinenlernverfahren ist, dass die Merkmale F nicht anfänglich vorgegeben sind, sondern vom Merkmalsextraktor 32 eigenständig ermittelt werden. Dieser untersucht hierzu das Sensorsignal S nach Mustern oder Regelmäßigkeiten und insbesondere nach Merkmalen F, welche in Kombination keinem schon vorbekannten Merkmalsvektor V entsprechen oder es wird explizit nach Merkmalen F gesucht, welche einem vorbekannten Merkmalsvektor V ähneln, um dessen Repräsentation zu verfeinern oder anzupassen.

In Fig. 3 ist weiterhin ein Zusatzsensor 34 gezeigt, welcher im Rahmen einer hybriden Analyse zusätzlich verwendet wird, um die Klassifikation des Sensorsignals S zu unterstützen oder um den Lernprozess zum Erlernen neuer Merkmalsvektoren N zu unterstützen. Der Zusatzsensor 34 liefert hierzu zusätzliche Informationen über die Umgebung. Der Zusatzsensor 34 ist beispielsweise eines der Mikrofone 8, welche jeweils ein Mikrofonsignal erzeugen, welches einer Audioanalyse unterzogen wird, um die aktuelle Situation zu klassifizieren und einen geeigneten Betriebsmodus auszuwählen. Nachdem dann für diese Situation die Muskelaktivität und ein entsprechender Merkmalsvektor N gelernt wurden, erfolgt eine zukünftige Erkennung entsprechend schneller und einfacher, ohne auf eine aufwendige Audioanalyse zurückgreifen zu müssen. Alternativ oder zusätzlich wird eine EEG-Messung durchgeführt, der Zusatzsensor 34 ist dann eine Anzahl an EEG-Elektroden. Geeignet ist auch ein Beschleunigungssensor als Zusatzsensor 34.

In den Fig. 1 bis 3 ist lediglich ein Einzelgerät eines Hörgeräts 2 gezeigt. In einer Variante weist das Hörgerät 2 jedoch zwei entsprechende Einzelgeräte auf, z.B. zwei Einzelgeräte jeweils wie in Fig. 2 gezeigt, welche dann entsprechend Fig. 1 auf unterschiedlichen Seiten des Kopfs des Anwenders getragen werden. Ein solches Hörgerät 2 wird dann als binaurales Hörgerät 2 bezeichnet. In dieser Konfiguration stehen also zwei Sensorsignale S zur Verfügung und somit insgesamt mehr Informationen, d.h. Merkmale F zur Klassifizierung der Intention des Anwenders und zur verbesserten Auswahl eines geeigneten Betriebsmodus.

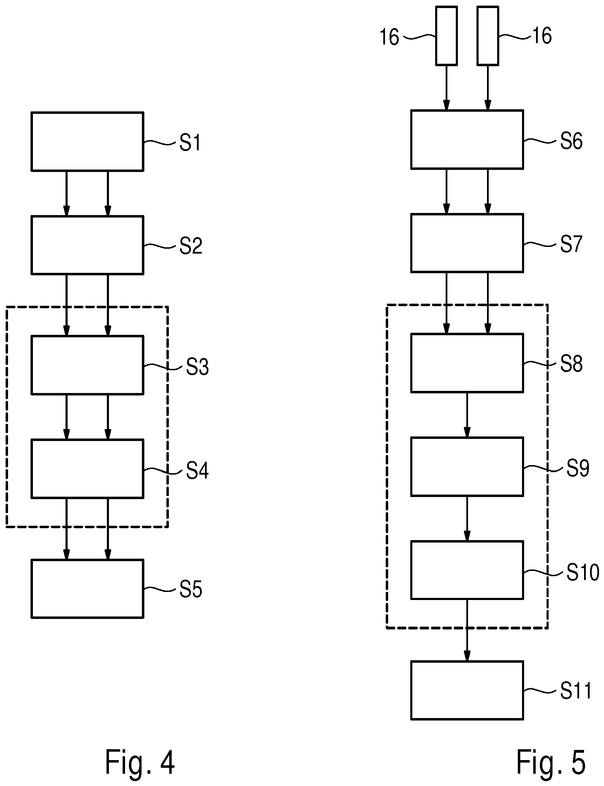

Ein beispielhafter Betrieb für ein solches binaurales Hörgerät 2 ist in den Fig. 4 und 5 gezeigt. Dabei wird eine Richtcharakteristik 24 des Hörgeräts 2 eingestellt, indem die Sensorsignale S die Elektrodenarrays 16 der beiden Einzelgeräte ausgewertet werden. Dabei zeigt Fig. 4 das Erlernen neuer Merkmalsvektoren N und die Anpassung der Filterbank 30 zur optimierten Erkennung von Merkmalen F im jeweiligen Sensorsignal S. In Fig. 5 ist schließlich die Anpassung der Richtcharakteristik 24 durch getrennte Auswertung der beiden Sensorsignale S dargestellt.

In Fig. 4 werden im ersten Schritt S1 die Sensorsignale S jeweils separat mittels EMG erzeugt. In Schritt S2 werden Rauschartefakte mittels des Vorfilters 26 entfernt. Die Schritte S3 und S4 betreffen nun die Anpassung und Einstellung der Merkmalserkennung und der Filterbank 30 im Rahmen des Maschinenlernverfahrens. Im Schritt S3 wird die parametrisierte Filterbank 30 optimiert, vor allem dahingehend, dass die beiden Sensorsignale S unterscheidbar sind, um jeweilige Merkmale F der rechten oder der linken Seite zuordnen zu können. In Schritt S4 wird ein neuer Merkmalsvektoren N extrahiert, um für den Klassifikator 20 als vorbekannter Merkmalsvektor V gespeichert. Im Schritt S5 ist dann das Hörgerät optimal auf den jeweiligen Anwender eingerichtet, d.h. personalisiert.

Fig. 5 zeigt nun, wie die vorbekannten Merkmalsvektoren V zur Klassifikation beim Richtungshören genutzt werden. Die Auswertung der Muskelaktivitäten der Ohrmuskeln M auf den beiden Seiten erfolgt zunächst getrennt, indem die Sensorsignale S unabhängig voneinander klassifiziert werden, und anschließend wird lediglich bei einem übereinstimmenden Ergebnis das Hörgerät 2 eingestellt. Es erfolgt also eine komparative Entscheidungsfindung. In Schritt S6 werden die Sensorsignale S mittels der Elektrodenarrays 16 erzeugt. In Schritt S7 werden die Sensorsignale S wie in Schritt S2 in Fig. 4 vorgefiltert. Die zuvor optimierte Filterbank 30 unterscheidet dann in Schritt S8 die beiden Sensorsignale S und bestimmt unabhängig voneinander deren Merkmale F. Es werden dann zwei Merkmalsvektoren G erzeugt, aus welchen in Schritt S9 durch Klassifikation auf eine bestimmte Intention geschlossen wird. Diese werden in Schritt S10 verglichen. Stimmen die Ergebnisse für beide Seiten überein, wird das Hörgerät 2 in Schritt S11 entsprechend eingestellt.

### Bezugszeichenliste

- 2: Hörgerät
- 4: Gehäuse
- 6: Schallschlauch
- 8: Mikrofon
- 10: Steuereinheit
- 12: Hörer
- 14: Ohrstück
- 16: Elektrodenarray
- 17: Elektrode
- 18: Signalverarbeitung
- 20: Klassifikator
- 22: Speicher
- 24: Richtcharakteristik
- 26: Vorfilter
- 28: Referenzelektrode
- 30: Filterbank
- 32: Merkmalsextraktor
- 34: Zusatzsensor

- F: Merkmal
- G: Merkmalsvektor
- M: Ohrmuskel
- N: neuer Merkmalsvektor
- O: Ohr
- S: Sensorsignal
- V: vorbekannter Merkmalsvektor

## Patentansprüche

1. Verfahren zum Betrieb eines Hörgeräts (2) für einen Anwender,
- wobei eine Elektromyographie durchgeführt wird, bei welcher mittels eines Elektrodenarrays (16) eine Muskelaktivität eines Ohrmuskels (M) des Anwenders gemessen wird,
- wobei unter "Ohrmuskel" einer der Muskeln der Ohrmuskulatur des Menschen verstanden wird, wobei die Ohrmuskulatur besteht aus den Muskeln auricularis anterior, auricularis posterior, auricularis superior, helicis major, helicis minor, transversus auriculae, obliquus auriculae, tragicus und antitragicus,
- wobei die Muskelaktivität fortlaufend gemessen wird, zur Erfassung eines komplexen Aktivitätsprofils des Ohrmuskels (M), in welchem eine Intention des Anwenders kodiert ist, wobei die Intention eine Hörintention ist,
- wobei bei der Messung der Muskelaktivität nicht lediglich ein Reflex gemessen wird,
- wobei das Elektrodenarray (16) ein Sensorsignal (S) erzeugt, welches mittels eines Klassifikators (20) einer Klassifikation unterzogen wird, bei welcher die Muskelaktivität dekodiert wird und die zugrunde liegende Intention bestimmt wird, indem untersucht wird, ob das Sensorsignal (S) einen vorbekannten Merkmalsvektor (V) aufweist,
- wobei dem vorbekannten Merkmalsvektor (V) ein Betriebsmodus des Hörgeräts (2) zugeordnet ist, welcher eingestellt wird, wenn das Sensorsignal (S) den vorbekannten Merkmalsvektor (V) aufweist.

2. Verfahren nach dem vorhergehenden Anspruch, wobei die Muskelaktivität über einen Messzeitraum von wenigstens 100ms, bevorzugt wenigstens 1s gemessen wird und dass der vorbekannte Merkmalsvektor (V) über den gesamten Messzeitraum in dem Sensorsignal (S) gesucht wird.

3. Verfahren nach einem der vorhergehenden Ansprüche, wobei das Hörgerät (2) zum Richtungshören ausgebildet ist und dass anhand der Muskelaktivität eine Richtcharakteristik (24) des Hörgeräts (2) eingestellt wird, wodurch eine Schallquelle in einer bestimmten Richtung hervorgehoben wird.

4. Verfahren nach einem der vorhergehenden Ansprüche, wobei das Hörgerät (2) ein binaurales Hörgerät (2) ist und zwei Einzelgeräte aufweist, zum Tragen auf unterschiedlichen Seiten des Kopfes des Anwenders, und dass auf jeder der beiden Seiten mittels eines Elektrodenarrays (16) ein Sensorsignal (S) erzeugt wird und eine Muskelaktivität eines dortigen Ohrmuskels (M) gemessen wird, die Muskelaktivitäten auf den beiden Seiten zunächst getrennt ausgewertet werden, indem die Sensorsignale (S) unabhängig voneinander klassifiziert werden, und anschließend bei einem übereinstimmenden Ergebnis das Hörgerät (2) eingestellt wird.

5. Verfahren nach einem der vorhergehenden Ansprüche, wobei das Hörgerät (2) ein binaurales Hörgerät (2) ist und zwei Einzelgeräte aufweist, zum Tragen auf unterschiedlichen Seiten des Kopfes des Anwenders, und auf jeder der beiden Seiten mittels eines Elektrodenarrays (16) ein Sensorsignal (S) erzeugt wird und eine Muskelaktivität eines dortigen Ohrmuskels (M) gemessen wird, die Muskelaktivitäten auf den beiden Seiten zunächst getrennt ausgewertet werden, indem die Sensorsignale (S) unabhängig voneinander klassifiziert werden, und bei unterschiedlichen Ergebnissen die Einzelgeräte synchronisiert werden, indem das eine der Einzelgeräte entgegen des eigenen Ergebnisses und anhand des Ergebnisses des anderen Einzelgeräts eingestellt wird.

6. Verfahren nach Anspruch 3, wobei das Hörgerät (2) ein binaurales Hörgerät (2) ist und zwei Einzelgeräte aufweist, zum Tragen auf unterschiedlichen Seiten des Kopfes des Anwenders, und auf jeder der beiden Seiten mittels eines Elektrodenarrays (16) ein Sensorsignal (S) erzeugt wird und eine Muskelaktivität eines dortigen Ohrmuskels (M) gemessen wird, die Intention eine gewollte Hörrichtung ist, welche ermittelt wird, indem untersucht wird, welches der beiden Sensorsignale (S) einen höheren Organisationsgrad oder eine höhere Energie aufweist, und die Richtcharakteristik (24) in Richtung der Hörrichtung eingestellt wird.

7. Verfahren nach einem der vorhergehenden Ansprüche, wobei anhand der Muskelaktivität eine Eigenstimmensituation erkannt wird, bei welcher der Anwender selbst spricht, und der Betriebsmodus eine Eigenstimmenreduktion ist, bei welcher ein Eigenstimmenanteil in einem Ausgangssignal des Hörgeräts (2) reduziert wird, und/oder anhand der Muskelaktivität ein Gesichtsausdruck des Anwenders erkannt wird, welcher als ein Steuerkommando zum Einstellen eines bestimmten Betriebsmodus verwendet wird, welcher dem Steuerkommando zugeordnet ist, und/oder anhand der Muskelaktivität eine Fehlpositionierung des Hörgeräts (2) erkannt wird, und/oder in Abhängigkeit der Muskelaktivität ein Verstärkungsschema, ein Kompressionsschema oder eine Rauschunterdrückung des Hörgeräts (2) eingestellt wird.

8. Verfahren nach einem der vorhergehenden Ansprüche, wobei das Sensorsignal (S) zusätzlich mittels eines Merkmalsextraktors (32) einer Merkmalsextraktion unterzogen wird, bei welcher ein neuer Merkmalsvektor (N) erzeugt wird, welcher als ein zusätzlicher vorbekannter Merkmalsvektor (V) gespeichert wird.

9. Verfahren nach Anspruch 8, wobei der neue Merkmalsvektor (N) in einem Maschinenlernverfahren mit Unterweisung erzeugt wird, indem
- einer bestimmten Situation ein bestimmter Betriebsmodus zugeordnet ist,
- die bestimmte Situation als die aktuelle Situation hergestellt,
- der Merkmalsextrator (32) eigenständig eine Anzahl an unbekannten Merkmalen (F) in dem Sensorsignal (S) sucht,
- die unbekannten Merkmale (F) zu dem neuen Merkmalsvektor (N) zusammengefasst werden,
- der neue Merkmalsvektor (N) dem bestimmten Betriebsmodus zugeordnet wird.

10. Verfahren nach einem der Ansprüche 8 oder 9, wobei ein Maschinenlernverfahren im Normalbetrieb des Hörgeräts (2) durchgeführt wird, wobei der Merkmalsextraktor (32) fortlaufend untersucht, ob im Sensorsignal (S) ein neuer Merkmalsvektor (N) enthalten ist und/oder mittels eines Zusatzsensors (34) erkannt wird, ob die aktuelle Situation eine vorbekannte Situation ist, welcher bereits ein vorbekannter Betriebsmodus zugeordnet ist, und der neue Merkmalsvektor (N) dem vorbekannten Betriebsmodus zugeordnet wird, falls die aktuelle Situation der vorbekannten Situation entspricht.

11. Verfahren nach einem der Ansprüche 8 bis 10, wobei der Merkmalsextraktor (32) und der Klassifikator (20) gemeinsam in eine Signalverarbeitung (18) integriert sind und die Merkmalsextraktion und die Klassifikation als ein einzelnes Optimierungsproblem behandelt werden.

12. Verfahren nach einem der vorhergehenden Ansprüche, wobei der vorbekannte Merkmalsvektor (V) in ein neuronales Netz integriert ist, mittels welchem untersucht wird, ob das Sensorsignal (S) einen vorbekannten Merkmalsvektor (V) aufweist, indem ein Merkmalsvektor des Sensorsignals (S) bestimmt wird und als ein Eingangssignal für das neuronale Netz verwendet wird, vorzugsweise mittels eines Zusatzsensors (34) erkannt wird, ob die aktuelle Situation eine vorbekannte Situation ist, welcher bereits ein vorbekannter Betriebsmodus zugeordnet ist, und vorzugsweise in das neuronale Netz ein Merkmalsvektor integriert ist, welcher dem vorbekannten Betriebsmodus zugeordnet wird, falls der integrierte Merkmalsvektor mit dem Merkmalsvektor des Sensorsignals (S) übereinstimmt.

13. Verfahren nach einem der vorhergehenden Ansprüche, wobei das Sensorsignal (S) mittels einer Filterbank (30) auf mehrere Frequenzbänder aufteilt wird, wobei jedes Frequenzband eine Anzahl an Merkmalen (F) bildet, die Filterbank (30) eine paraunitäre und parametrisierte Filterbank (30) ist, die Filterbank (30) vorzugsweise eine untere Grenzfrequenz von 4Hz aufweist und eine obere Grenzfrequenz von 1kHz, sodass die Muskelaktivität in einem Frequenzbereich von 4Hz bis 1kHz untersucht wird.

14. Verfahren nach einem der vorhergehenden Ansprüche, wobei das Elektrodenarray (16) höchstens fünf, bevorzugt genau zwei Elektroden (17, 28) aufweist, vorzugsweise eine der Elektroden (17, 28) als eine Referenzelektrode (28) ausgebildet ist, mittels welcher ein Referenzsignal abseits jeglicher Ohrmuskeln (M) des Anwenders gemessen wird, und mittels des Referenzsignals das Sensorsignal (S) aufbereitet wird, bevor dieses dem Klassifikator (20) zugeführt wird.

15. Hörgerät (2), welches eine Steuereinheit (10) aufweist, welche zur Durchführung eines Verfahrens nach einem der vorhergehenden Ansprüche ausgebildet ist.

## Claims

1. Method of operating a hearing device (2) for a user,
- wherein an electromyography is carried out, in which a muscle activity of an ear muscle (M) of the user is measured by means of an electrode array (16),
- wherein "ear muscle" is understood to be one of the muscles of the human ear musculature, wherein the ear musculature consists of the muscles auricularis anterior, auricularis posterior, auricularis superior, helicis major, helicis minor, transversus auriculae, obliquus auriculae, tragicus and antitragicus,
- wherein muscle activity is measured continuously, to capture a complex activity profile of the ear muscle (M), in which a user intention is encoded, wherein the intention being a hearing intention,
- wherein the measurement of muscle activity does not merely measure a reflex,
- wherein the electrode array (16) generates a sensor signal (S), which is classified by means of a classifier (20), in which the muscle activity is decoded and the underlying intention is determined by examining whether the sensor signal (S) comprises a previously known feature vector (V),
wherein an operating mode of the hearing device (2) is assigned to the previously known feature vector (V), which is set when the sensor signal (S) comprises the previously known feature vector (V).

2. Method according to the preceding claim,
wherein
the muscle activity is measured over a measurement period of at least 100ms, preferably at least 1s, and the previously known feature vector (V) is searched for in the sensor signal (S) over the entire measurement period.

3. Method according to one of the preceding claims,
wherein
the hearing device (2) is configured for directional hearing and a directivity (24) of the hearing device (2) is set based on the muscle activity, whereby a sound source is emphasized in a specific direction.

4. Method according to one of the preceding claims,
wherein
the hearing device (2) is a binaural hearing device (2) and comprises two individual devices to be worn on different sides of the head of the user, and on each of the two sides a sensor signal (S) is generated by means of an electrode array (16) and a muscle activity of a local ear muscle (M) is measured,
the muscle activities on both sides are first evaluated separately by classifying the sensor signals (S) independently of each other, and
subsequently, if the results are identical, the hearing device (2) is set.

5. Method according to one of the preceding claims,
wherein
the hearing device (2) is a binaural hearing device (2) and comprises two individual devices to be worn on different sides of the head of the user, and on each of the two sides a sensor signal (S) is generated by means of an electrode array (16) and a muscle activity of a local ear muscle (M) is measured,
the muscle activities on both sides are first evaluated separately by classifying the sensor signals (S) independently of each other, and
if the results differ, the individual devices are synchronized by setting one of the individual devices against its own result and based on the result of the other individual device.

6. Method according to claim 3,
wherein
the hearing device (2) is a binaural hearing device (2) and comprises two individual devices to be worn on different sides of the head of the user, and on each of the two sides a sensor signal (S) is generated by means of an electrode array (16) and a muscle activity of a local ear muscle (M) is measured,
the underlying intention is a desired hearing direction, which is determined by examining which of the two sensor signals (S) has a higher degree of organization or a higher energy, and
the directivity (24) is set towards the hearing direction.

7. Method according to one of the preceding claims,
wherein
based on the muscle activity, an own-voice situation is recognized, in which the user himself speaks, and the operating mode is an own-voice reduction, in which an own-voice component in an output signal of the hearing device (2) is reduced,
and/or
based on the muscle activity, a facial expression of the user is recognized, which is used as a control command for setting a specific operating mode associated with the control command,
and/or
based on the muscle activity a faulty positioning of the hearing device (2) is detected,
and/or
based on the muscle activity, an amplification scheme, a compression scheme or noise suppression of the hearing device (2) is set.

8. Method according to one of the preceding claims,
wherein
the sensor signal (S) is additionally subjected to a feature extraction by means of a feature extractor (32), in which a new feature vector (N) is generated, which is stored as an additional previously known feature vector (V).

9. Method according to claim 8,
wherein
- the new feature vector (N) is generated in a supervised machine learning process by
- associating a particular situation with a particular operating mode,
- establishing the particular situation as the current situation,
- the feature extractor (32) independently searching a number of unknown features (F) in the sensor signal (S),
- combining the unknown features (F) to form the new feature vector (N),
- assigning the new feature vector (N) to the particular operating mode.

10. Method according to one of claims 8 or 9,
wherein
a machine learning process is carried out during normal operation of the hearing device (2), wherein the feature extractor (32) continuously examines whether a new feature vector (N) is contained in the sensor signal (S) and/or
by means of an additional sensor (34) it is detected whether the current situation is a previously known situation to which a previously known operating mode is already assigned, and the new feature vector (N) is assigned to the previously known operating mode if the current situation corresponds to the previously known situation.

11. Method according to one of claims 8 to 10,
wherein
the feature extractor (32) and the classifier (20) are integrated together into a signal processor (18) and the feature extraction and classification are treated as a single optimization problem.

12. Method according to one of the preceding claims,
wherein
the previously known feature vector (V) is integrated into a neural network, by means of which it is examined whether the sensor signal (S) comprises a previously known feature vector (V) by determining a feature vector of the sensor signal (S) and using it as an input signal for the neural network, preferably by means of an additional sensor (34) it is detected whether the current situation is a previously known situation to which a previously known operating mode is already assigned, and
preferably a feature vector is integrated into the neural network, which is assigned to the previously known operating mode, if the integrated feature vector matches the feature vector of the sensor signal (S).

13. Method according to one of the preceding claims,
wherein
the sensor signal (S) is divided into a plurality of frequency bands by means of a filter bank (30), wherein each frequency band forms a number of features (F),
the filter bank (30) is a paraunitary and parameterized filter bank (30),
the filter bank (30) preferably has a lower cut-off frequency of 4Hz and an upper cut-off frequency of 1kHz, so that the muscle activity is investigated in a frequency range from 4Hz to 1kHz.

14. Method according to any of the preceding claims,
wherein
the electrode array (16) comprises at most five, preferably exactly two electrodes (17, 28),
preferably one of the electrodes (17, 28) is designed as a reference electrode (28), by means of which a reference signal is measured away from any ear muscles (M) of the user, and the sensor signal (S) is processed by means of the reference signal before it is fed to the classifier (20).

15. Hearing device (2) comprising a control unit (10) adapted to perform a method according to one of the preceding claims.

## Revendications

1. Procédé pour le fonctionnement d'une aide auditive (2) pour un utilisateur,
- dans lequel une électromyographie est effectuée, dans laquelle au moyen d'un réseau d'électrodes (16) l'activité d'un muscle de l'oreille (M) de l'utilisateur est mesurée,
- dans lequel le "muscle de l'oreille" est l'un des muscles de la musculature de l'oreille humaine, dans lequel la musculature de l'oreille est constituée des muscles auricularis anterior, auricularis posterior, auricularis superior, helicis major, helicis minor, transversus auriculae, obli-quus auriculae, tragicus et antitragicus,
- dans lequel l'activité musculaire est mesurée en continu, pour la détection d'un profil d'activité complexe du muscle de l'oreille (M), dans lequel est codée une intention de l'utilisateur, dans lequel l'intention est une intention auditive,
- dans lequel la mesure de l'activité musculaire ne se limite pas à la mesure d'un réflexe,
- dans lequel le réseau d'électrodes (16) génère un signal de capteur (S), qui est soumis au moyen d'un classificateur (20) à une classification dans laquelle l'activité musculaire est décodée et l'intention sous-jacente est déterminée en examinant si le signal de capteur (S) comprend un vecteur de caractéristique (V) connu précédemment,
- dans lequel un mode de fonctionnement de l'aide auditive (2) est attribué au vecteur de caractéristique (V) connu précédemment, dans lequel ledit mode de fonctionnement est réglé lorsque le signal du capteur (S) comprend le vecteur de caractéristique (V) connu précédemment.

2. Procédé selon la revendication précédente,
dans lequel
l'activité musculaire est mesurée sur une période de mesure d'au moins 100 ms, de préférence d'au moins 1 s, et le vecteur de caractéristique (V) précédemment connu est recherché dans le signal du capteur (S) sur toute la période de mesure.

3. Procédé selon l'une des revendications précédentes,
dans lequel
l'aide auditive (2) est conçue pour l'audition directionnelle et une directivité (24) de l'aide auditive (2) est réglée sur la base de l'activité musculaire, ce qui permet d'accentuer une source sonore dans une direction spécifique.

4. Procédé selon l'une des revendications précédentes,
dans lequel
l'aide auditive (2) est une aide auditive binaurale (2) et comprend deux dispositifs individuels à porter sur des côtés différents de la tête de l'utilisateur, et sur chacun des deux côtés un signal de capteur (S) est généré au moyen d'un réseau d'électrodes (16) et une activité musculaire d'un muscle de l'oreille (M) est mesurée,
les activités musculaires des deux côtés sont d'abord évaluées séparément en classant les signaux des capteurs (S) indépendamment les uns des autres, et si les résultats correspondent. l'aide auditive (2) est alors réglée

5. Procédé selon l'une des revendications précédentes,
dans lequel
l'aide auditive (2) est une aide auditive binaurale (2) et comprend deux dispositifs individuels à porter sur des côtés différents de la tête de l'utilisateur et sur chacun des deux côtés un signal de capteur (S) est généré au moyen d'un réseau d'électrodes (16) et une activité musculaire d'un muscle de l'oreille local (M) est mesurée,
les activités musculaires des deux côtés sont d'abord évaluées séparément en classant les signaux des capteurs (S) indépendamment les uns des autres, et si les résultats sont différents, les différents appareils sont synchronisés en réglant l'un des appareils individuels contre son propre résultat et en fonction du résultat de l'autre appareil individuel.

6. Procédé selon la revendication 3,
dans lequel
l'aide auditive (2) est une aide auditive binaurale (2) et comprend deux dispositifs individuels à porter sur des côtés différents de la tête de l'utilisateur et sur chacun des deux côtés un signal de capteur (S) est généré au moyen d'un réseau d'électrodes (16) et une activité musculaire d'un muscle de l'oreille local (M) est mesurée,
l'intention sous-jacente est une direction d'audition souhaitée, qui est déterminée en examinant lequel des deux signaux de capteur (S) comprend un degré d'organisation ou une énergie plus élevée, et
la directivité (24) est réglée vers la direction de l'audition.

7. Procédé selon l'une des revendications précédentes,
dans lequel
l'activité musculaire est utilisée pour détecter une situation de propre voix dans laquelle l'utilisateur parle lui-même, et le mode de fonctionnement est un mode de réduction de la propre voix, dans laquelle une composante de la propre voix dans un signal de sortie de l'aide auditive (2) est réduite, et/ou
sur la base de l'activité musculaire, une expression faciale de l'utilisateur est reconnue, qui est utilisée comme commande de contrôle pour régler un mode de fonctionnement spécifique associé à la commande de contrôle, et/ou
sur la base de l'activité musculaire, un mauvais positionnement de l'aide auditive (2) est détecté,
et/ou
sur la base de l'activité musculaire, un schéma d'amplification, un schéma de compression ou de suppression du bruit de l'aide auditive (2) est réglé.

8. Procédé selon l'une des revendications précédentes,
dans lequel
le signal du capteur (S) est en outre soumis à une extraction de caractéristiques au moyen d'un extracteur de caractéristique (32), dans laquelle un nouveau vecteur de caractéristique (N) est généré, qui est stocké comme un vecteur de caractéristique supplémentaire connu précédemment (V).

9. Procédé selon la revendication 8,
dans lequel
le nouveau vecteur de caractéristique (N) est généré dans un procédé d'apprentissage machine supervisé en
- associant une situation spécifique à un mode de fonctionnement spécifique,
- établissant la situation spécifique comme étant la situation actuelle,
- l'extracteur de caractéristique (32) recherchant indépendamment un certain nombre de caractéristique inconnues (F) dans le signal du capteur (S),
- en combinant les caractéristiques inconnues (F) pour former le nouveau vecteur de caractéristique (N),
- attribuant le nouveau vecteur de caractéristique (N) au mode de fonctionnement spécifique.

10. Procédé selon l'une des revendications 8 ou 9,
dans lequel
un procédé d'apprentissage machine est effectué pendant le fonctionnement normal de l'aide auditive (2), dans lequel l'extracteur de caractéristique (32) vérifie en continu si un nouveau vecteur de caractéristique (N) est contenu dans le signal du capteur (S)
et/ou
au moyen d'un capteur supplémentaire (34), il est détecté si la situation actuelle est une situation connue précédemment, à laquelle un mode de fonctionnement connu précédemment est déjà attribué, et le nouveau vecteur de caractéristique (N) est attribué au mode de fonctionnement connu précédemment si la situation actuelle correspond à la situation connue précédemment.

11. Procédé selon l'une des revendications 8 à 10,
dans lequel
l'extracteur de caractéristique (32) et le classificateur (20) sont intégrés ensemble dans un traitement de signal (18) et l'extraction de caractéristique et la classification sont traitées comme un seul problème d'optimisation.

12. Procédé selon l'une des revendications précédentes,
dans lequel
le vecteur de caractéristique (V) connu précédemment est intégré dans un réseau neuronal, au moyen duquel il est examiné si le signal du capteur (S) comprend un vecteur de caractéristique (V) connu précédemment en déterminant un vecteur de caractéristique du signal du capteur (S) et en utilisant ledit vecteur de caractéristique (V) comme signal d'entrée pour le réseau neuronal,
de préférence au moyen d'un capteur supplémentaire (34), il est détecté si la situation actuelle est une situation connue précédemment à laquelle un mode de fonctionnement connu précédemment est déjà attribué, et
de préférence un vecteur de caractéristique est intégré dans le réseau neuronal, qui est attribué au mode de fonctionnement connu précédemment si le vecteur de caractéristique intégré correspond au vecteur de caractéristique du signal du capteur (S).

13. Procédé selon l'une des revendications précédentes,
dans lequel
le signal du capteur (S) est divisé en plusieurs bandes de fréquences au moyen d'un banc de filtres (30), dans lequel chaque bande de fréquences forme un certain nombre de marqueurs (F),
la banque de filtres (30) est une banque de filtres para-unitaires et paramétrés (30),
la banque de filtres (30) comprend de préférence une fréquence de coupure inférieure de 4 Hz et une fréquence de coupure supérieure de 1 kHz, de sorte que l'activité musculaire est examinée dans une gamme de fréquences entre 4 Hz et 1 kHz.

14. Procédé selon l'une des revendications précédentes,
dans lequel
le réseau d'électrodes (16) comporte au maximum cinq, de préférence exactement deux électrodes (17, 28),
de préférence, l'une des électrodes (17, 28) est conçue comme une électrode de référence (28), au moyen de laquelle un signal de référence est mesuré à distance de tout muscle de l'oreille (M) de l'utilisateur, et le signal du capteur (S) est traité au moyen du signal de référence avant d'être envoyé au classificateur (20).

15. Aide auditive (2), comprenant une unité de contrôle (10), qui est conçue pour effectuer un procédé selon l'une des revendications précédentes.
